# EUROPÄISCHE PATENTANMELDUNG

(11) **EP 3 088 406 A1**
(43) Veröffentlichungstag der Anmeldung: **02.11.2016**
(21) Anmeldenummer: 16165358.9
(22) Anmeldetag: 14.04.2016
(51) Int. Cl.: C07F 9/6574, B01J 31/22

(54) **NEUE MONOPHOSPHITVERBINDUNGEN MIT EINER ETHERGRUPPE**

(30) Priorität: 29.04.2015 DE 102015207864
(71) Anmelder: Evonik Degussa GmbH, 45128 Essen (DE)
(72) Erfinder: Dyballa, Katrin Marie, 45657 Recklinghausen (DE); Franke, Robert, 45772 Marl (DE)

(57) **Zusammenfassung**

Neue Monophosphitverbindungen mit einer Ethergruppe, sowie ein Verfahren zur Herstellung dieser Verbindungen, die insbesondere zur Verwendung als Liganden in Hydroformylierungsreaktionen geeignet sind.

## Beschreibung

Die vorliegende Erfindung betrifft neue Monophosphitverbindungen mit einer Ethergruppe sowie ein Verfahren zur Herstellung der Verbindungen, die insbesondere zur Verwendung als Liganden in Hydroformylierungsreaktionen geeignet sind. Die Erfindung betrifft weiterhin auch Komplexe, die zumindest eine dieser Monophosphitverbindungen aufweisen sowie die Verwendung der vorgenannten Verbindungen und/oder Komplexe in Hydroformylierungsreaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}.

Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

In Angew. Chem. Int. Ed. 2000, 39, No. 9, S.1639-1641 von Börner et al. werden Liganden beschrieben, welche eine P-C- und zwei P-O-Bindungen aufweisen, es handelt sich somit um Phosphonite. Die dort beschriebenen Phosphonite weisen bei einem Einsatz in der Hydroformylierung n/iso-Selektivitäten (n/iso = das Verhältnis von linearem Aldehyd (= n) zu verzweigtem (= iso) Aldehyd)) von 0,61 bis 1,57 auf.

Die in DE 199 54 721 A1 beschriebenen Phosphonitliganden weisen eine gute n/iso-Selektivität auf. Allerdings haben Untersuchungen ergeben, dass die Verbindung II-c (in DE 199 54 721; Seite 6) zu einer photochemisch induzierten Zersetzung neigt. Aufgrund der Instabilität der Verbindung ist diese nicht lagerfähig, weshalb die Verwendung der Verbindung als Ligand in der Katalyse großtechnisch nicht wirtschaftlich ist. Von einem großtechnischen Einsatz der Verbindung ist daher abzusehen.

Ein weiterer Nachteil von Liganden mit einer Phosphonitstruktur besteht in der sehr aufwändigen Herstellung. Die Möglichkeit einer günstigen und einfachen Synthese spielt jedoch für den Einsatz von Liganden in einem großtechnischen Prozess eine elementare Rolle, da die Produktionskosten für einen Liganden nur so hoch sein dürfen, dass die Rentabilität des Gesamtprozesses, in dem der Ligand später eingesetzt wird, weiterhin gewährleistet bleibt.

Rhodium-Monophosphit-Komplexe in katalytisch aktiven Zusammensetzungen sind geeignet für die Hydroformylierung von verzweigten Olefinen mit innenständigen Doppelbindungen.

Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt. Hierbei werden jedoch z.T. sehr hohe Rhodiumkonzentrationen verwendet (u.a. 250 ppm), was in Anbetracht des derzeitigen Rhodiumpreises für ein großtechnisches Verfahren nicht wirtschaftlich ist.

Gegenüber dem bekannten Stand der Technik ist es die Aufgabe der vorliegenden Erfindung, alternative Liganden und diese aufweisende Komplexe zum Einsatz in Hydroformylierungsreaktionen bereitzustellen, die die im Zusammenhang mit den vorbekannten Liganden aufgezeigten Nachteile nicht aufweisen. Insbesondere sollen die neuen Liganden vorzugsweise mit geringerem Aufwand und entsprechend auch mit geringeren Kosten synthetisierbar sein als die aus dem Stand der Technik bekannten Liganden. Bevorzugt sollen die neuen Liganden auch in der großtechnischen Hydroformylierung einsetzbar sein.

Diese Aufgabe wird gelöst durch Verbindungen gemäß Anspruch 1. Insbesondere wird die Aufgabe gelöst durch Monophosphitverbindungen, die eine der beiden allgemeinen Strukturen I oder II aufweisen oder daraus bestehen: 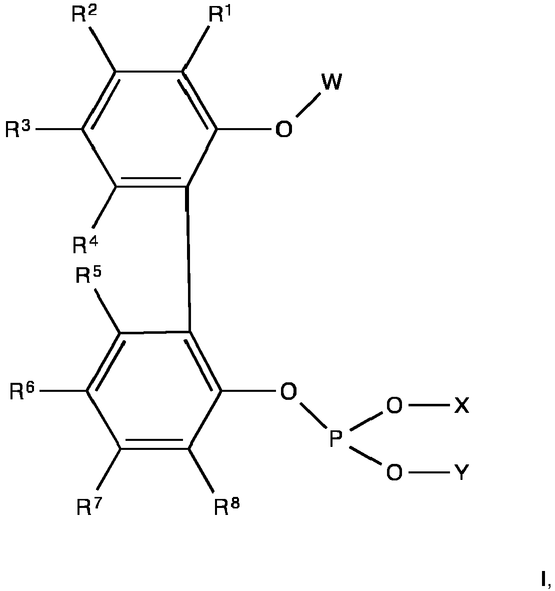 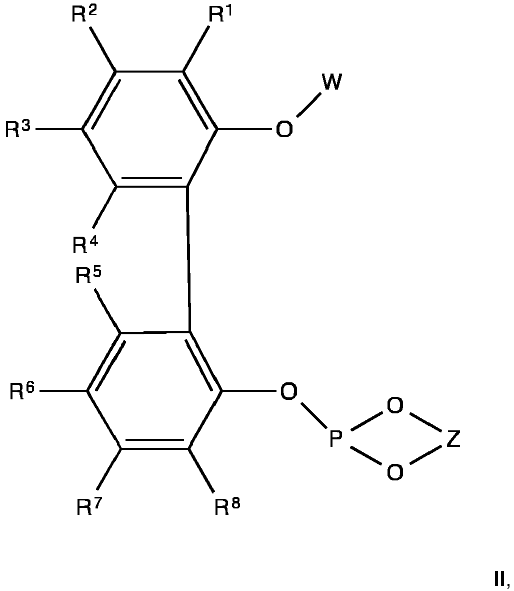
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM), -CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-*c*-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, -CH₂C₄H₆-*p*-NO₂, -CH₂C₆H₃-2,6-Cl₂, -CH₂C₆H₃,4-Cl₂, -CH₂-9-anthyl, -CH₂-4-pyridyl, -Si(CH₃)₃, -Si(CH₃)₂C(CH₃)₃;
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₁-C₁₂)-Alkyl-(C₆-C₂₀)-Aryl,-(C₆-C₂₀)-Aryl-COO-(C₁-C₁₂)-Alkyl,-(C₆-C₂₀)-Aryl-CONH-(C₁-C₁₂)-Alkyl,-(C₆-C₂₀)-Aryl-CON[(C₁-C₁₂)-Alkyl]₂, -(C₄-C₂₀)-Heteroaryl, -(C₄-C₂₀)-Heteroaryl-(C₁-C₁₂)-Alkyl, -(C₅-C₈)-Cycloalkyl, -(C₅-C₈)-Heterocycloalkyl, -( C₆-C₂₀)-Aryl-CO-(C₆-C₂₀)-Aryl;
und wobei Z ausgewählt ist aus: -(C₁-C₁₂)-Alkyl-, -( C₆-C₂₀)-Aryl-, -(C₆-C₂₀)-Aryl-(C₁-C₁₂)-Alkyl-, -( C₁-C₁₂)-Alkyl-( C₆-C₂₀)-Aryl-, -(C₄-C₂₀)-Heteroaryl-, -(C₆-C₂₀)-Aryl-CO-(C₆-C₂₀)-Aryl-, -(C₆-C₂₀)-Aryl-(C₆-C₂₀)-Aryl-.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₃-C₁₂)-Cycloalkyl und (C₃-C₁₂)-Heterocycloalkyl können jeweils unsubstituiert oder durch einen oder mehrere gleiche oder verschiedene Reste substituiert sein, die ausgewählt sind unter (C₁-C₁₂)-Alkyl, (C₁-C₁₂)-Alkoxy, (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl.

(C₆-C₂₀)-Aryl und -(C₆-C₂₀)-Aryl-(C₆-C₂₀)-Aryl- können jeweils unsubstituiert oder durch einen oder mehrere gleiche oder verschiedene Reste substituiert sein, die ausgewählt sind unter -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, ido-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge einen oder mehrere Substituenten aufweisen. Die Substituenten sind vorzugsweise unabhängig voneinander ausgewählt unter -(C₃-C₁₂)-Cycloalkyl, - (C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, - N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Substituierte -(C₃-C₁₂)-Cycloalkylgruppen und substituierte -(C₃-C₁₂)-Heterocycloalkylgruppen können, in Abhängigkeit von ihrer Ringgröße, einen oder mehrere (z.B. 1, 2, 3, 4 oder 5) weitere Substituenten aufweisen. Diese Substituenten sind vorzugsweise unabhängig voneinander ausgewählt unter -(C₁-C₁₂)-Alkyl, -(C₁-C₁₂)-Alkoxy, -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl. Substituierte -(C₃-C₁₂)-Cycloalkylgruppen tragen vorzugsweise eine oder mehrere -(C₁-C₆)-Alkylgruppen. Substituierte - (C₃-C₁₂)-Heterocycloalkylgruppen tragen vorzugsweise eine oder mehrere -(C₁-C₆)-Alkylgruppen.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter -H, - (C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise einen oder mehrere, z.B. 1, 2, 3, 4 oder 5 Substituenten, ausgewählt unter -(C₁-C₁₂)-Alkylgruppen, -(C₁-C₁₂)-Alkoxygruppen.

Obwohl in den Verbindungen gemäß den Formeln I und II generell alle vorgenannten Substituenten mögliche Substituenten für die als R¹ bis R⁸ bezeichneten Reste sind, handelt es sich bei -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CO-(C₁-C₁₂)-Alkyl, -CO-(C₆-C₂₀)-Aryl, -COOH, -OH, -SO₃H, -NO₂, -CN, -NH₂, -N[(C₁-C₁₂)-Alkyl]₂ um bevorzugte Substituenten für R¹ bis R⁸. Insbesondere sind als Substituenten für R¹ bis R⁸ bevorzugt - H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -OH, -NO₂, -NH₂.

In einer Ausführungsform sind R¹ bis R⁸ bevorzugt -H, -(C₁-C₁₂)-Aikyi, -O-(C₁-C₁₂)-Alkyl, -O-(C₆-C₂₀)-Aryl, -(C₆-C₂₀)-Aryl, -Cl, -F, -Br, -I.

In einer Ausführungsform sind R¹ bis R⁸ bevorzugt -H, -(C₁-C₁₂)-Alkyl, -O-(C₁-C₁₂)-Alkyl, -O-(C₆-C₂₀)-Aryl, -(C₆-C₂₀)-Aryl, besonders bevorzugt davon sind -H, -(C₁-C₁₂)-Alkyl und -O-(C₁-C₁₂)-Alkyl.

In einer Ausführungsform stehen R³ und R⁶ für -O-(C₁-C₁₂)-Alkyl.

In einer Ausführungsform stehen R³ und R⁶ für -OMe.

In einer Ausführungsform stehen R¹ und R⁸ für -(C₁-C₁₂)-Alkyl.

In einer Ausführungsform stehen R¹ und R⁸ für *tert*-Butyl.

In einer Ausführungsform stehen R¹, R³, R⁶ und R⁸ für -(C₁-C₁₂)-Alkyl.

In einer Ausführungsform stehen R¹, R³, R⁶ und R⁸ für Methyl.

In einer Ausführungsform stehen R¹, R³, R⁶ und R⁸ für *tert*-Butyl.

In einer Ausführungsform stehen R¹, R², R³, R⁴, R⁵, R⁶, R⁷ und R⁸ für -H.

In einer Ausführungsform sind bevorzugte Substituenten für W ausgewählt unter: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM), -2-tetrahydropyranyl, -CH(OC₂H₅)CH₃, -CH₂COC₆H₅, -CH₂-*c*-C₃H₅, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, -CH₂C₄H₆-*p*-NO₂, -CH₂-9-anthyl, -CH₂-4-pyridyl, -Si(CH₃)₃, -Si(CH₃)₂C(CH₃)₃;

In einer Ausführungsform sind bevorzugte Substituenten für W ausgewählt unter: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -2-tetrahydropyranyl, -CH(OC₂H₅)CH₃, -CH₂COC₆H₅, -CH₂-*c*-C₃H₅, - CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃,-CH₂-9-anthyl,-CH₂-4-pyridyl.

In einer Ausführungsform sind bevorzugte Substituenten für W ausgewählt unter: -(C₁-C₁₂)-Alkyl, -Me, -CH₂CH₃, -CH₂CH₂CH₃, -CH₂-*c*-C₃H₅, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃,-CH₂-9-anthyl, -CH₂-4-pyridyl.

In einer Ausführungsform sind bevorzugte Substituenten für W ausgewählt unter: -CH₂OCH₃ (MOM), -CH₂OCH₂C₆H₅ (BOM), -CH₂OCH₂CH₂OCH₃ (MEM), -2-tetrahydropyranyl, - CH(OC₂H₅)CH₃, -CH₂COC₆H₅.

In einer Ausführungsform sind bevorzugte Substituenten für W ausgewählt unter: -Me, -CH₂CH₃, -CH₂CH₂CH₃, -CH₂-*c*-C₃H₅, -CH(CH₃)₂, -*c*-C₆H₁₁, -C(CH₃)₃, -CH₂C₆H₅, -CH₂C₆H₃-2,4-(CH₃)₂.

Von den vorgenannten Substituenten X und Y in Verbindungen gemäß Formel I sind insbesondere - (C₁-C₁₂)-Alkyl, -(C₆-C₂₀)-Aryl, -(C₆-C₂₀)-Aryl-(C₁-C₁₂)-Alkyl, -(C₁-C₁₂)-Alkyl-(C₆-C₂₀)-Aryl, -(C₄-C₂₀)-Heteroaryl und -(C₅-C₈)-Cycloalkyl als Substituenten bevorzugt.

In einer Ausführungsform stehen X und Y für die gleichen Reste.

Von den vorgenannten möglichen Substituenten für Z in Verbindungen gemäß Formel II sind -(C₁-C₁₂)-Alkyl-, -(C₆-C₂₀)-Aryl-, -(C₆-C₂₀)-Aryl-(C₁-C₁₂)-Alkyl-, -(C₁-C₁₂)-Alkyl-(C₆-C₂₀)-Aryl-und -(C₆-C₂₀)-Aryl-(C₆-C₂₀)-Aryl- als Substituenten bevorzugt. Besonders bevorzugte Substituenten Z weisen zumindest einen Phenylring und vorzugsweise zwei oder optional mehr Phenylringe auf, wobei die Phenylringe optional substituiert sind, insbesondere mit (C₁-C₁₂)-Alkylresten oder mit -O-(C₁-C₁₂)-Alkylresten.

In einer Ausführungsform steht Z für:

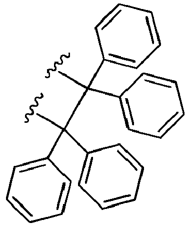

In einer weiteren Ausführungsform steht Z für -(C₆-C₂₀)-Aryl-(C₆-C₂₀)-Aryl-.

In einer Ausführungsform weist die Verbindung die allgemeine Struktur **III** auf: 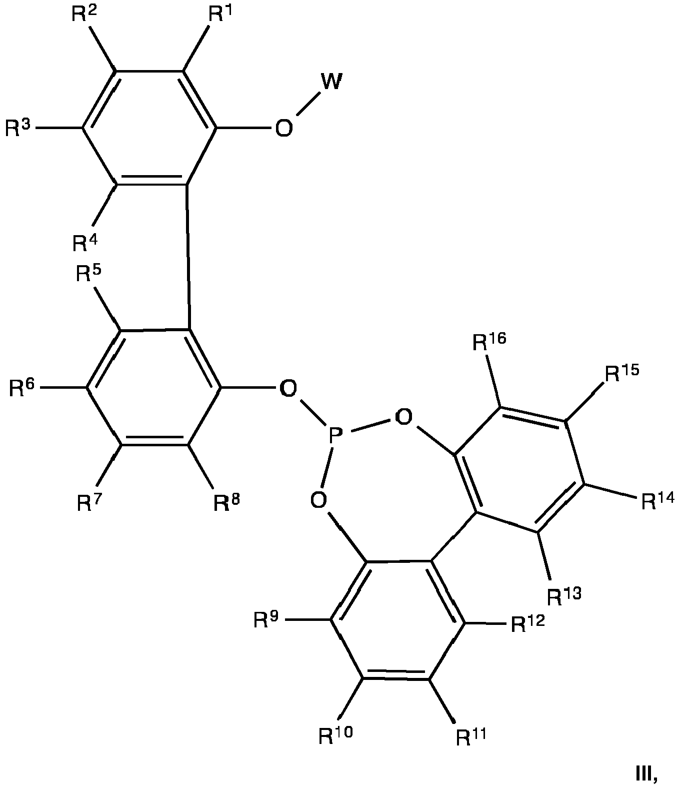
wobei R¹, R², R³, R⁴, R⁵, R⁶, R⁷, R⁸ und W so definiert sind wie im Zusammenhang mit Verbindungen gemäß Formeln I und II angegeben,
und wobei R⁹, R¹⁰, R¹¹, R¹², R¹³, R¹⁴, R¹⁵, R¹⁶ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), - COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

In einer Ausführungsform sind R⁹, R¹⁰, R¹¹, R¹², R¹³, R¹⁴, R¹⁵, R¹⁶ jeweils unabhängig voneinander ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl,-COOH, - OH, -NH₂, -N[(C₁-C₁₂)-Alkyl]₂.

In einer Ausführungsform sind R⁹, R¹⁰, R¹¹, R¹², R¹³, R¹⁴, R¹⁵, R¹⁶ jeweils unabhängig voneinander ausgewählt aus: -H, -(C₁-C₁₂)-Alkyl, -O-(C₁-C₁₂)-Alkyl, -O-(C₆-C₂₀)-Aryl, -(C₆-C₂₀)-Aryl, -COO-(C₁-C₁₂)-Alkyl, - N[(C₁-C₁₂)-Alkyl]₂.

In einer Ausführungsform sind R⁹, R¹⁰, R¹¹, R¹², R¹³, R¹⁴, R¹⁵, R¹⁶ jeweils unabhängig voneinander ausgewählt aus:
-H, -(C₁-C₁₂)-Alkyl, -O-(C₁-C₁₂)-Alkyl, -(C₆-C₂₀)-Aryl,
wobei Alkyl 1-12 Kohlenstoffatome, bevorzugt 1-10 Kohlenstoffatome z.B. primäre, sekundäre oder tertiäre Alkylgruppe, wie Methyl-, Ethyl-, n-Propyl-, Isopropyl-, Butyl-, sec-Butyl-, t-Butyl-, t-Butylethyl-, t-Butylpropyl-, n-Hexyl-, Amyl-, sec-Amyl-, t-Amyl-, iso-Octyl-, 2-Ethylhexyl-, Decyl-, Dodecyl- und Octadecylgruppen umfasst.

In einer Ausführungsform stehen R¹¹ und R¹⁴ für -O-(C₁-C₁₂)-Alkyl.

In einer Ausführungsform stehen R¹¹ und R¹⁴ für-OMe.

In einer Ausführungsform stehen R⁹ und R¹⁶ für -(C₁-C₁₂)-Alkyl.

In einer Ausführungsform stehen R⁹ und R¹⁶ für *tert*-Butyl.

In einer Ausführungsform stehen R⁹, R¹¹, R¹⁴ und R¹⁶ für -(C₁-C₁₂)-Alkyl.

In einer Ausführungsform stehen R⁹, R¹¹, R¹⁴ und R¹⁶ für Methyl.

In einer Ausführungsform stehen R⁹, R¹¹, R¹⁴ und R¹⁶ für *tert*-Butyl.

In einer Ausführungsform stehen R⁹, R¹⁰, R¹¹, R¹², R¹³, R¹⁴, R¹⁵ und R¹⁶ für -H.

In bevorzugten Ausführungsformen weisen erfindungsgemäße Monophosphitverbindungen eine der acht folgenden allgemeinen Strukturen **IV**, **V**, **VI, VII, VIII, IX, X** oder **XI** auf:

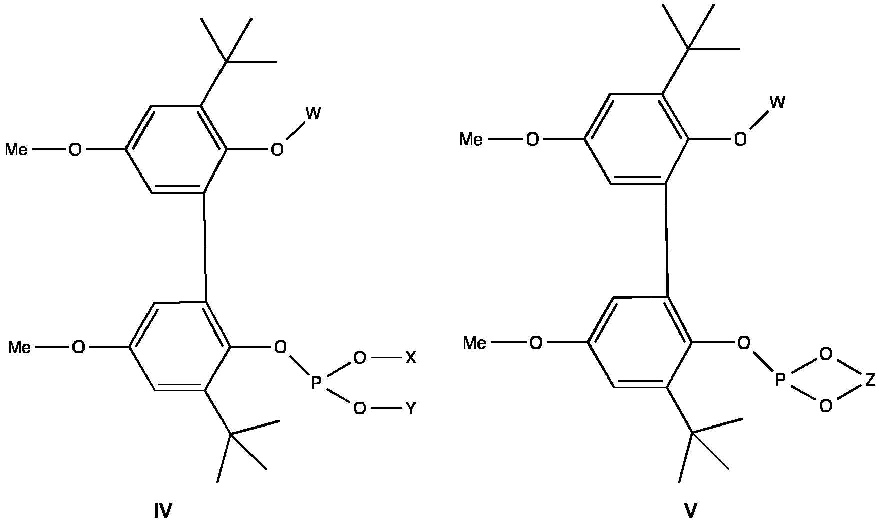

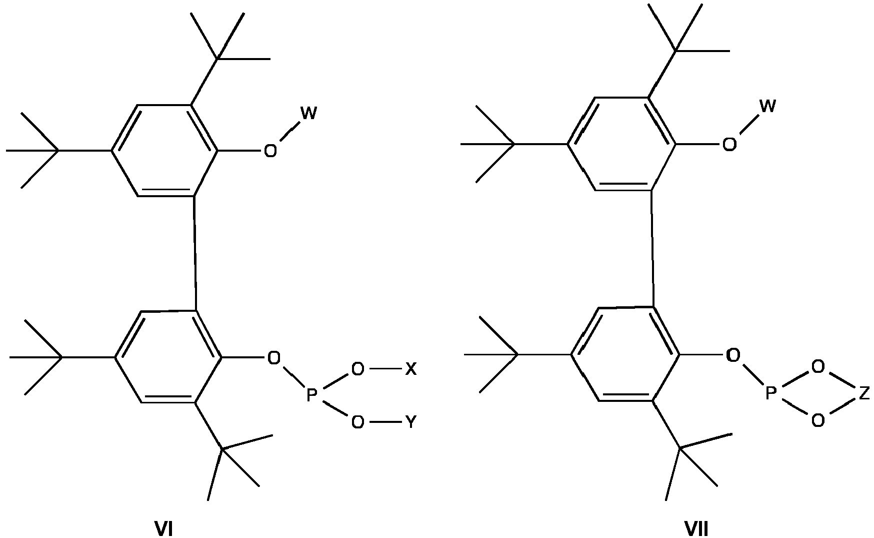

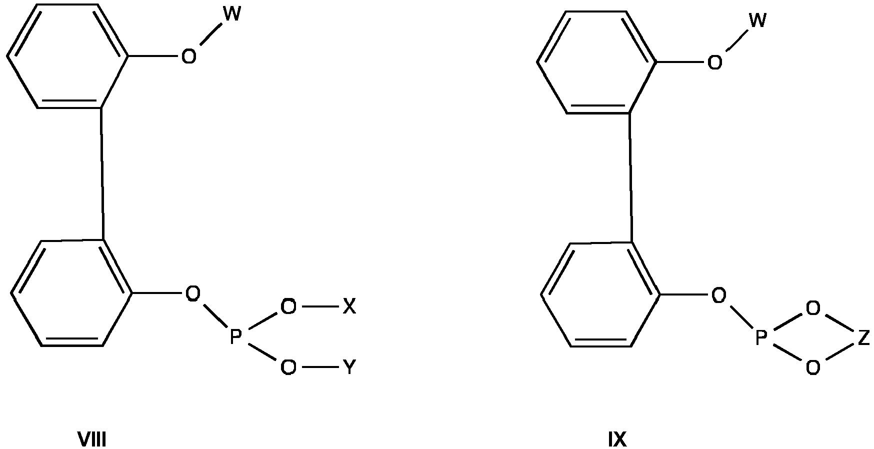

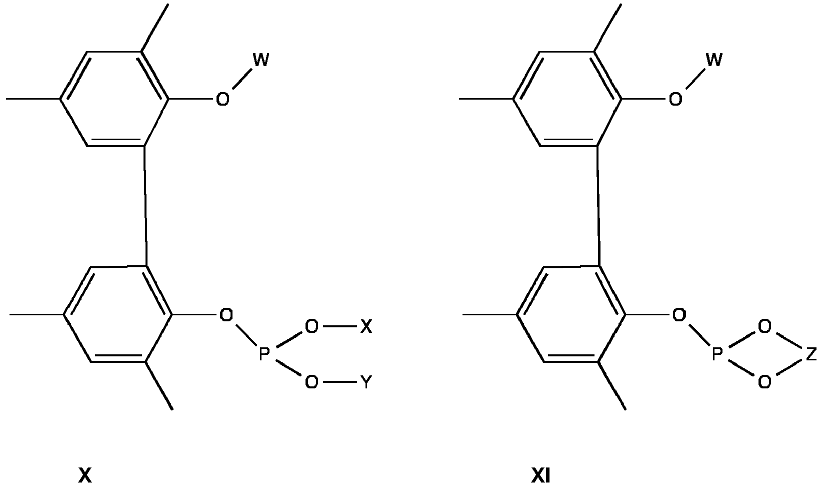

Besonders bevorzugte erfindungsgemäße Verbindungen weisen beispielsweise eine der nachfolgenden Strukturen **L1, L2** oder **L3** auf:

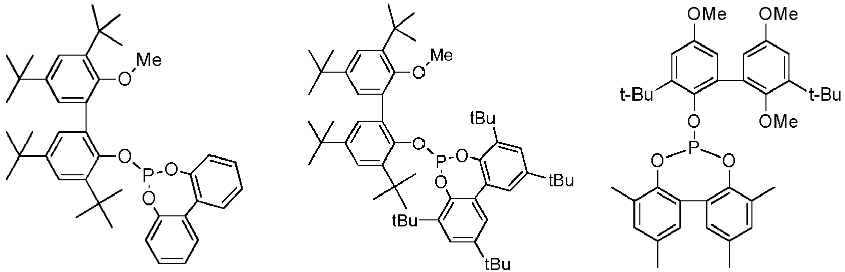

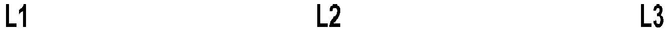

Die erfindungsgemäßen Monophosphitverbindungen zeichnen sich insbesondere durch eine gute Stabilität und damit verbunden durch eine gute Lagerfähigkeit aus, insbesondere sind die erfindungsgemäßen Monophosphitverbindungen stabiler und einfacher zu synthetisieren als die aus der DE 199 54 721 A1 bekannten Phosphonite.

Die Erfindung betrifft weiterhin auch ein Verfahren zur Herstellung von Verbindungen, welche eine der beiden allgemeinen Strukturen **I** oder **II** aufweisen: 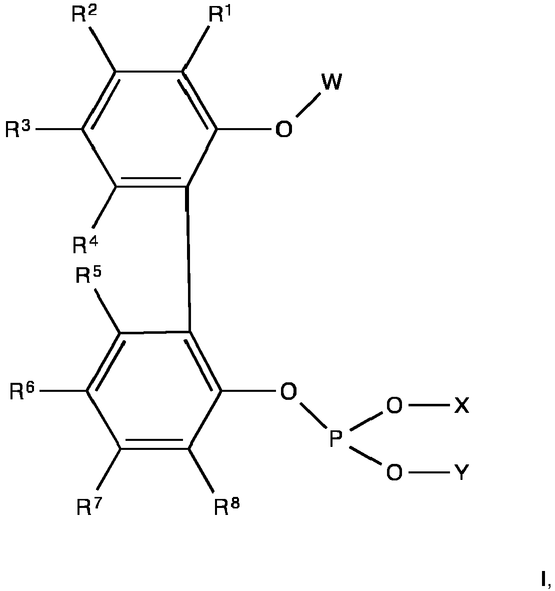 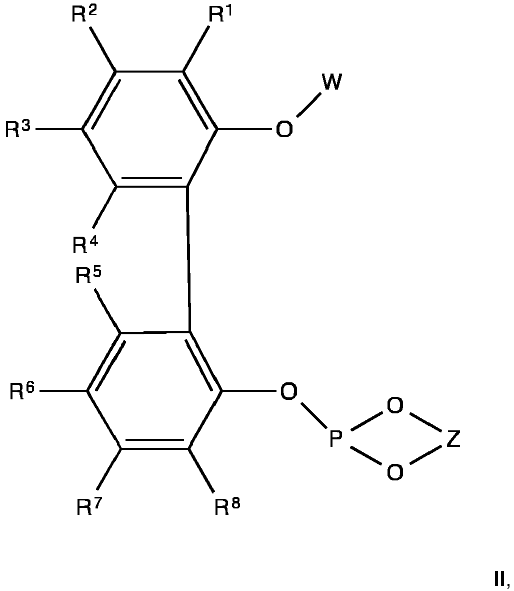
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM), -CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-*c*-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, -CH₂C₄H₆-*p*-NO₂, -CH₂C₆H₃-2,6-Cl₂, -CH₂C₆H₃-3,4-Cl₂, -CH₂-9-anthyl, -CH₂-4-pyridyl, -Si(CH₃)₃, -Si(CH₃)₂C(CH₃)₃;
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₁-C₁₂)-Alkyl-(C₆-C₂₀)-Aryl,-(C₆-C₂₀)-Aryl-COO-(C₁-C₁₂)-Alkyl,-(C₆-C₂₀)-Aryl-CONH-(C₁-C₁₂)-Alkyl,-(C₆-C₂₀)-Aryl-CON[(C₁-C₁₂)-Alkyl]₂, -(C₄-C₂₀)-Heteroaryl, -(C₄-C₂₀)-Heteroaryl-(C₁-C₁₂)-Alkyl, -(C₅-C₈)-Cycloalkyl, -(C₅-C₈)-Heterocycloalkyl, -(C₆-C₂₀)-Aryl-CO-(C₆-C₂₀)-Aryl;
und wobei Z ausgewählt ist aus: -(C₁-C₁₂)-Alkyl-, -(C₆-C₂₀)-Aryl-, -(C₆-C₂₀)-Aryl-(C₁-C₁₂)-Alkyl-, -(C₁-C₁₂)-Alkyl-(C₆-C₂₀)-Aryl-, -(C₄-C₂₀)-Heteroaryl-, -(C₆-C₂₀)-Aryl-CO-(C₆-C₂₀)-Aryl-, -(C₆-C₂₀)-Aryl-(C₆-C₂₀)-Aryl-;
und wobei die genannten Alkyl-, Heteroalkyl-, Cycloalkyl-, Heterocycloalkyl-, Aryl- und Heteroarylgruppen optional substituiert sind, insbesondere jeweils unabhängig voneinander mit -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, - CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, - COOH, -OH, -SO₃H; -SO₃Na, -NO₂, -CN, -NH₂; -N[(C₁-C₁₂)-Alkyl]₂.

Das erfindungsgemäße Verfahren zur Herstellung von Monophosphitverbindungen weist dabei die folgenden Schritte a) - d) auf, deren Reihenfolge nicht notwendigerweise der Reihenfolge ihrer Nennung entsprechen muss:
a) Vorlegen eines Eduktes,
b) Zugabe eines Alkylierungsreagenzes,
c) Zugabe einer Verbindung, die Phosphor und Chlor enthält oder daraus besteht, und
d) Erhalten eines Produktes.

Für die Zwecke der Erfindung umfasst der Begriff Alkylierungsreagenz ebenfalls ein Silylierungsreagenz. Aufgrund der Vereinfachung sind beide Begriffe unter Alkylierungsreagenz zusammengefasst.

Exemplarisch werden im Folgenden drei Ausführungsformen des erfindungsgemäßen Herstellungsverfahrens in vereinfachter Form dargestellt. Es wird darauf hingewiesen, dass die in der Beschreibung der einzelnen Ausführungsformen des Verfahrens beispielhaft erwähnten Basen und Alkylierungsreagenzien durch andere, dem Fachmann bekannte Basen und Alkylierungsreagenzien ersetzt werden können, und dass nach Bedarf in allen Schritten zusätzlich Base verwendet werden kann.

Die erste Ausführungsform des erfindungsgemäßen Verfahrens zur Herstellung von Verbindungen mit einer Struktur gemäß Formel **I** oder **II** umfasst im Wesentlichen drei Synthesestufen, die in den Reaktionsschemata 1a und 1b dargestellt sind. In Reaktionsschema 1a ist die Herstellung von Monophosphitverbindungen mit einer Struktur gemäß Formel **I** dargestellt:

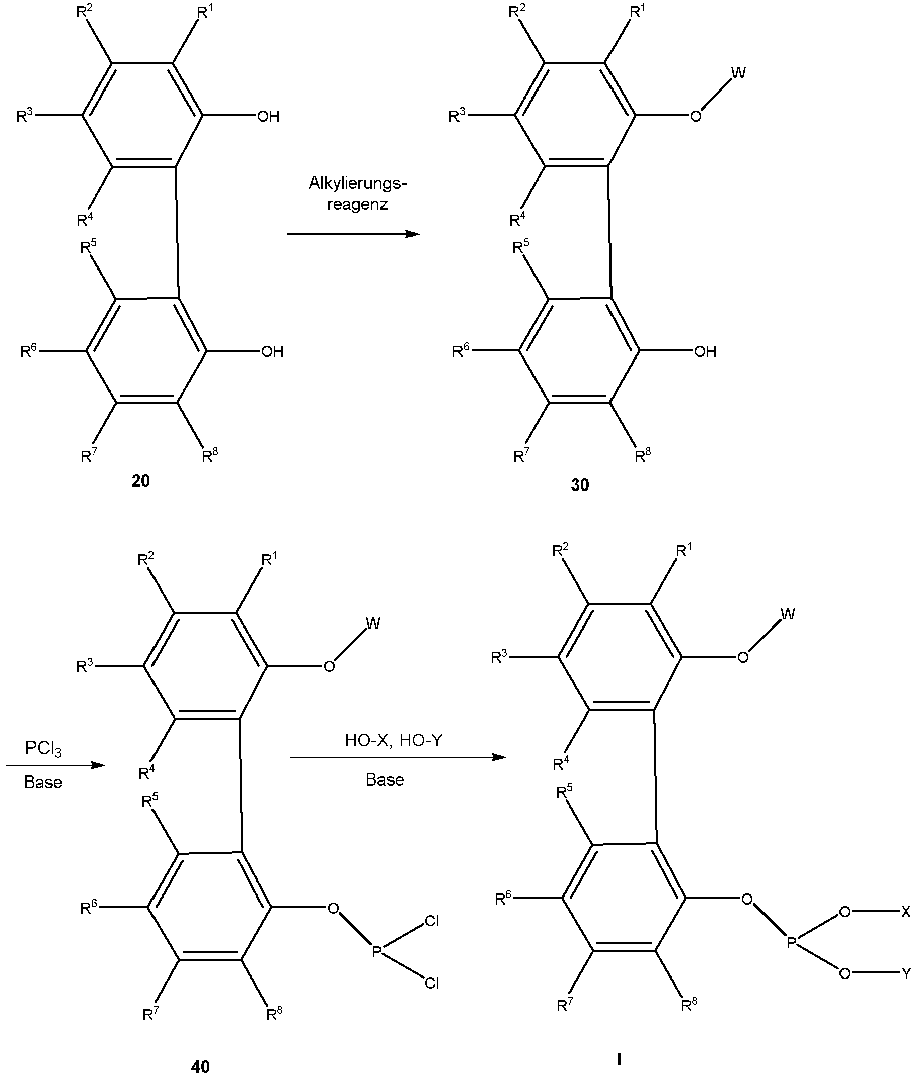

### Reaktionsschema 1a: Darstellung erfindungsgemäßer Monophosphitverbindungen mit einer Struktur gemäß Formel I gemäß einer ersten Ausführungsform des Verfahrens

In der ersten Stufe der in Reaktionsschema 1a gezeigten Synthese von Verbindungen mit einer Struktur gemäß Formel **I** erfolgt eine Veretherung der in para-Stellung zu dem Substituenten R³ befindlichen Hydroxylgruppe eines Biphenol-Derivates **20** durch Reaktion mit einem Alkylierungsreagenz, z.B. mit Dimethylsulfat oder Mel, wodurch die Verbindung **30** erhalten wird. In der zweiten Synthesestufe wird PCl₃ zu der veretherten Verbindung **30** gegeben, welches in Anwesenheit einer Base, z.B. Triethylamin, mit der nicht-alkylierten Hydroxylgruppe (in para-Stellung zu dem Substituenten R⁶) unter HCl-Abspaltung bzw. unter Bildung des entsprechenden Aminhydrochlorids, das durch Reaktion der Base mit dem freiwerdenden HCl gebildet wird, zum Chlorophosphit **40** reagiert. In der dritten Synthesestufe erfolgt durch Reaktion mit den Alkoholen HO-X und HO-Y ebenfalls unter HCl-Abspaltung bzw. unter Bildung des durch Reaktion der Base, z.B. Triethylamin, mit dem freiwerdenden HCl gebildeten Aminhydrochlorids die Umsetzung zur Monophosphitverbindung gemäß Formel **I.**

In Reaktionsschema 1 b ist die Herstellung von Monophosphitverbindungen mit einer Struktur gemäß Formel II dargestellt:

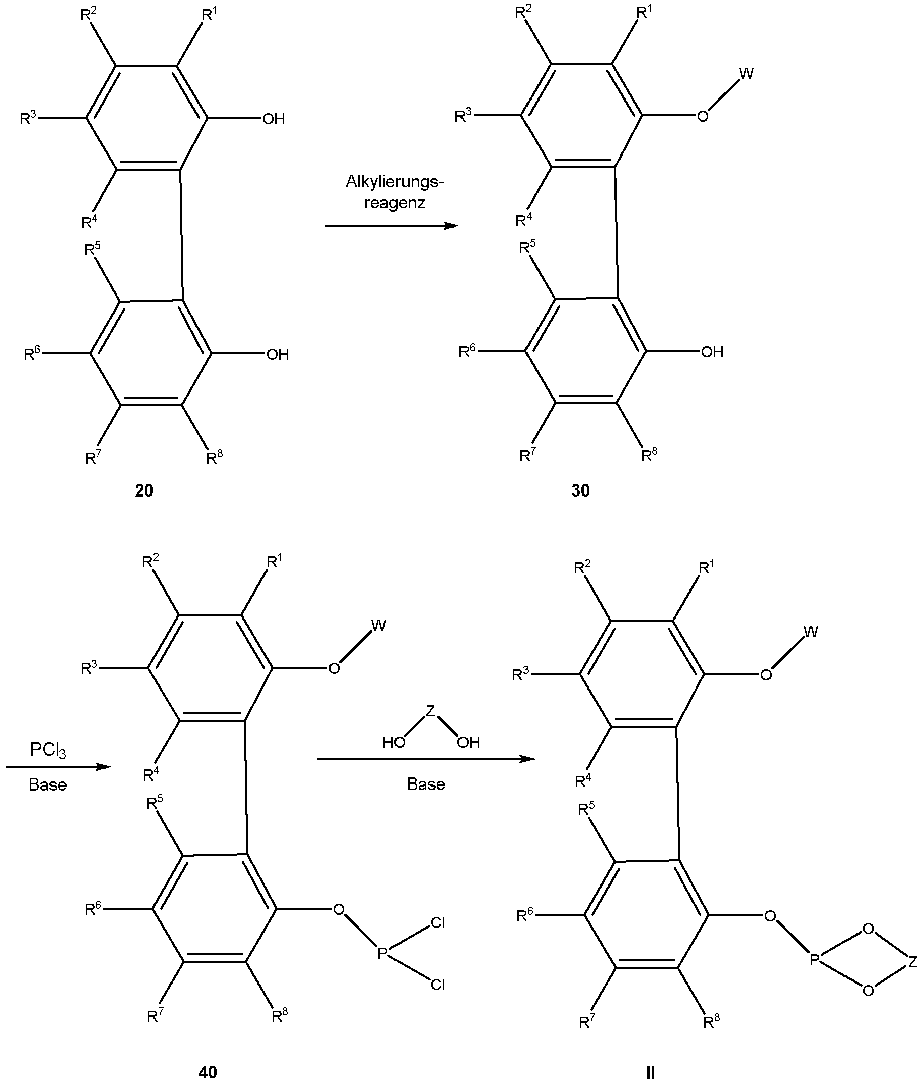

### Reaktionsschema 1b: Darstellung erfindungsgemäßer Monophosphitverbindungen mit einer Struktur gemäß Formel II gemäß einer ersten Ausführungsform des Verfahrens

Die in Reaktionsschema 1 b gezeigte Synthese von Verbindungen mit einer Struktur gemäß Formel **II** erfolgt bis zur Herstellung des Chlorophosphits **40** identisch zu der in Reaktionsschema 1a gezeigten Synthese von Verbindungen mit einer Struktur gemäß Formel **I**. Das Chlorophosphit **40** wird dann jedoch abweichend von der in Reaktionsschema 1a gezeigten Synthese nicht mit den Alkoholen HO-X und HO-Y, sondern mit einem die Gruppe Z aufweisenden Diol zur Monophosphitverbindung mit einer Struktur gemäß Formel **II** umgesetzt.

Zusammenfassend zeichnet sich das erfindungsgemäße Verfahren in der ersten Ausführungsform also durch das Vorhandensein des Chlorophosphits **40** als Zwischenstufe aus.

Die zweite Ausführungsform des erfindungsgemäßen Verfahrens zur Herstellung von Verbindungen mit einer Struktur gemäß Formel **I** oder **II** umfasst im Wesentlichen zwei Synthesestufen. In Reaktionsschema 2a ist die Herstellung von Monophosphitverbindungen mit einer Struktur gemäß Formel **I** dargestellt, während in Reaktionsschema 2b die Herstellung von Monophosphitverbindungen mit einer Struktur gemäß Formel **II** dargestellt ist.

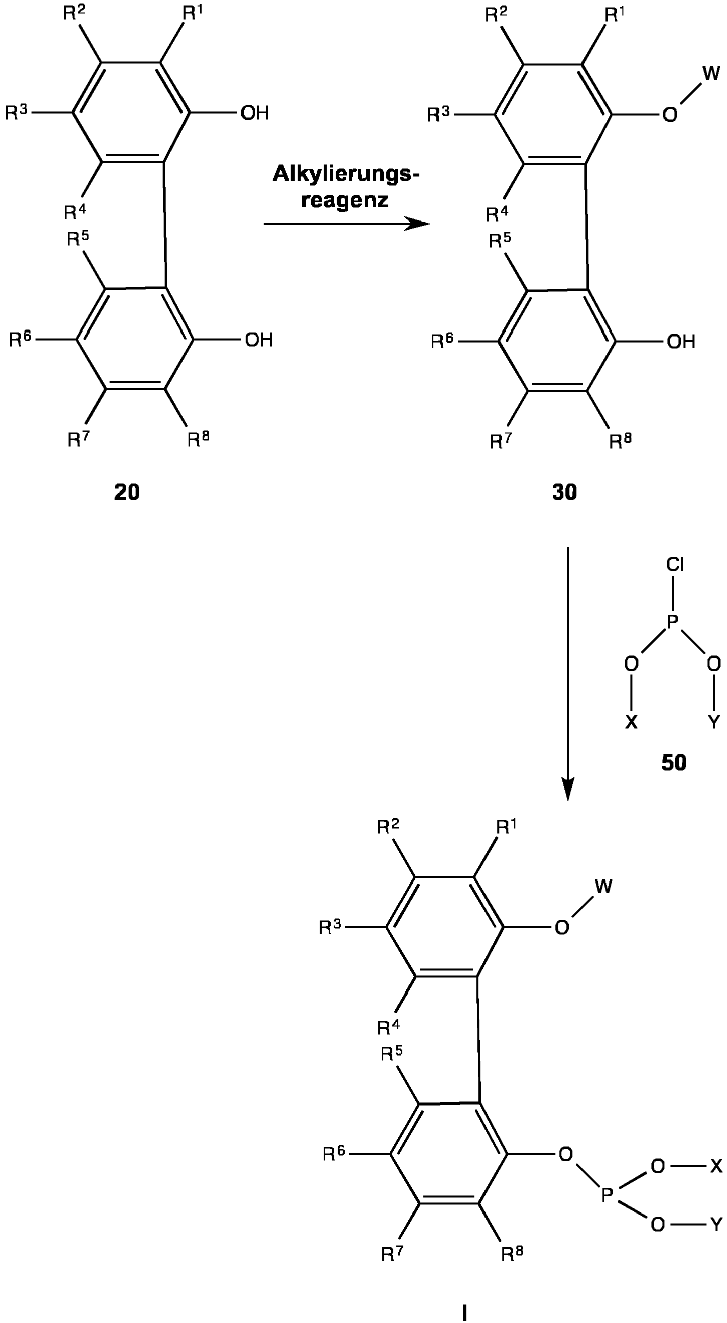

### Reaktionsschema 2a: Darstellung erfindungsgemäßer Monophosphitverbindungen mit einer Struktur gemäß Formel I gemäß einer zweiten Ausführungsform des Verfahrens

Gemäß der in Reaktionsschema 2a gezeigten Ausführungsform des erfindungsgemäßen Verfahrens erfolgt in der ersten Synthesestufe zunächst eine Veretherung einer Hydroxylgruppe eines Biphenol-Derivates **20** durch Reaktion mit einem Alkylierungsreagenz, z.B. Dimethylsulfat oder Mel. Dieser Syntheseschritt entspricht dem der ersten Synthesestufe gemäß der in den Reaktionsschemata 1a und 1b gezeigten ersten Ausführungsform des erfindungsgemäßen Verfahrens. Das veretherte Reaktionsprodukt **30** der ersten Synthesestufe wird nun im Gegensatz zu der ersten Ausführungsform des erfindungsgemäßen Verfahrens nicht zu einem Chlorophosphit umgesetzt, sondern mit einem Chlorophosphit, nämlich dem Chlorophosphit der allgemeinen Struktur **50**. Synthesewege zur Herstellung von Chlorophosphiten sind dem Fachmann hinlänglich bekannt, darüber hinaus wird an späterer Stelle die Synthese ausgewählter Chlorophosphite beschrieben. Als Reaktionsprodukt der zweiten Synthesestufe entsteht eine erfindungsgemäße Monophosphitverbindung mit einer Struktur gemäß Formel **I.**

Erfindungsgemäße Monophosphitverbindungen mit einer Struktur gemäß Formel **II** sind gemäß der zweiten Ausführungsform des erfindungsgemäßen Verfahrens entsprechend des in Reaktionsschema 2b dargestellten Syntheseweges erhältlich.

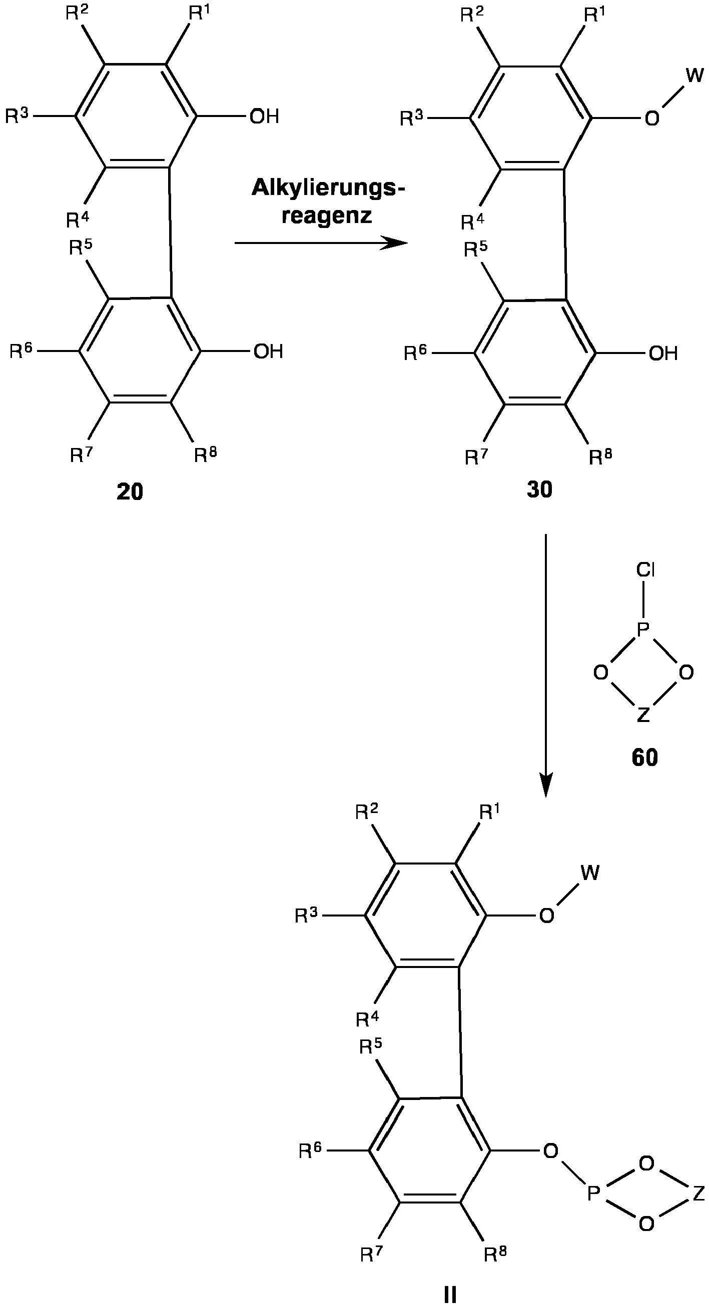

### Reaktionsschema 2b: Darstellung erfindungsgemäßer Monophosphitverbindungen mit einer Struktur gemäß Formel II gemäß einer zweiten Ausführungsform des Verfahrens

Die erste Synthesestufe des in Reaktionsschema 2b gezeigten Verfahrens ist die bereits aus dem Reaktionsschema 2a bekannte Veretherung der Verbindung **20** zu dem Zwischenprodukt **30.** In der zweiten Synthesestufe erfolgt wie auch auf dem in Reaktionsschema 2a gezeigten Reaktionsweg die Umsetzung der Verbindung **30** mit einem Chlorophosphit. Dieses hat jedoch eine von dem in der Reaktion gemäß Reaktionsschema 2a verwendeten Chlorophosphit abweichende Struktur, nämlich die Struktur gemäß der allgemeinen Formel **60**, sodass als Reaktionsprodukt die Monophosphitverbindung mit einer Struktur gemäß Formel **II** entsteht. Wie bereits im Zusammenhang mit dem in Reaktionsschema 2a gezeigten Syntheseweg erwähnt, sind dem Fachmann Wege zur Herstellung von Chlorophosphiten hinlänglich bekannt. Die Herstellung ausgewählter Chlorophosphite wird darüber hinaus an späterer Stelle beschrieben.

Zusammenfassend zeichnet sich das erfindungsgemäße Verfahren gemäß der zweiten Ausführungsform dadurch aus, dass es die Umsetzung eines Biphenol-Derivates **20** mit einem Chlorophosphit der allgemeinen Struktur **50** oder **60** einschließt.

Gemäß einer dritten Ausführungsform des erfindungsgemäßen Verfahrens zur Herstellung von Verbindungen, die eine Struktur gemäß Formel **I** oder **II** aufweisen, erfolgt die Veretherung, die gemäß der ersten beiden Ausführungsformen jeweils die erste Synthesestufe des Verfahrens gebildet hat, erst in der letzten Synthesestufe.

In Reaktionsschema 3a ist die Herstellung von Monophosphitverbindungen mit einer Struktur gemäß Formel **I** gemäß der dritten Ausführungsform des Verfahrens dargestellt::

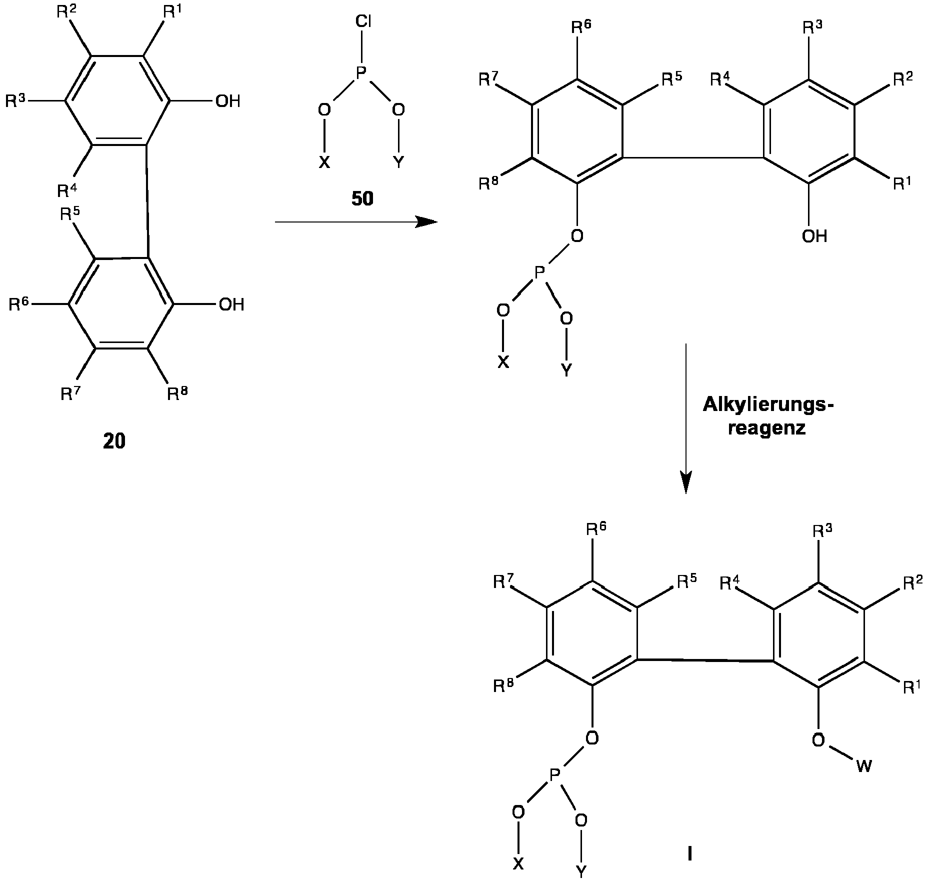

### Reaktionsschema 3a: Darstellung erfindungsgemäßer Monophosphitverbindungen mit einer Struktur gemäß Formel I gemäß einer dritten Ausführungsform des Verfahrens

In der ersten Stufe der in Reaktionsschema 3a gezeigten Synthese von Verbindungen mit einer Struktur gemäß Formel **I** wird ein Biphenol-Derivat **20** mit einem Chlorophosphit **50** zu einer Monophosphitverbindung umgesetzt, die sich von einer erfindungsgemäßen Monophosphitverbindung mit einer Struktur gemäß Formel **I** nur dadurch unterscheidet, dass sie in para-Stellung zu dem Substituenten R³ anstelle einer über ein Sauerstoffatom gebundenen Alkylgruppe eine Hydroxylgruppe aufweist. In der zweiten Synthesestufe erfolgt dann die Veretherung in para-Stellung zu dem Substituenten R³, z.B. durch Reaktion mit Dimethylsulfat oder Mel, wodurch eine Verbindung mit einer Struktur gemäß Formel **I** erhalten wird.

Für Monophosphitverbindungen gemäß Formel **II** ist der Syntheseweg gemäß der dritten Ausführungsform in Reaktionsschema 3b dargestellt:

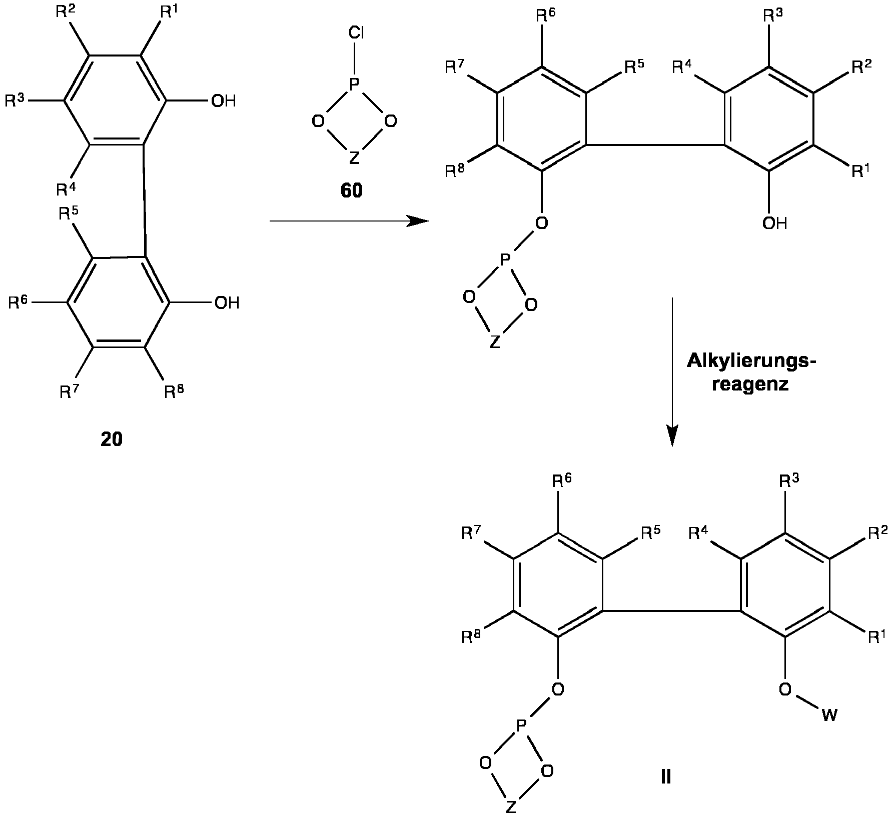

### Reaktionsschema 3b: Darstellung erfindungsgemäßer Monophosphitverbindungen mit einer Struktur gemäß Formel II gemäß einer dritten Ausführungsform des Verfahrens

In der ersten Stufe der in Reaktionsschema 3b gezeigten Synthese von Verbindungen mit einer Struktur gemäß Formel **II** wird ein Biphenol-Derivat **20** mit einem Chlorophosphit **60** zu einer Monophosphitverbindung umgesetzt, die sich von einer erfindungsgemäßen Monophosphitverbindung mit einer Struktur gemäß Formel **II** nur dadurch unterscheidet, dass sie in para-Stellung zu dem Substituenten R³ anstelle einer über ein Sauerstoffatom gebundenen Alkylgruppe eine Hydroxylgruppe aufweist. In der zweiten Synthesestufe erfolgt dann die Veretherung in para-Stellung zu dem Substituenten R³, z.B. durch Reaktion mit Dimethylsulfat oder Mel, wodurch eine Verbindung mit einer Struktur gemäß Formel **II** erhalten wird.

Die Substituenten R¹, R², R³, R⁴, R⁵, R⁶, R⁷, R⁸, W, X, Y und Z sind in der vorliegenden Anmeldung nicht in jeder gezeigten allgemeinen Struktur neu definiert. Es ist aber beabsichtigt, dass die Substituenten R¹, R², R³, R⁴, R⁵, R⁶, R⁷, R⁸, W, X, Y und Z in jeder allgemeinen Struktur, in der sie enthalten sind, identisch zu der im Zusammenhang mit den Formeln **I** und **II** gegebenen Definition definiert sind.

Die Erfindung betrifft außerdem die Verwendung einer Verbindung mit einer Struktur gemäß Formel **I** oder **II** als Ligand in einem Ligand-Metall-Komplex zur Katalyse von Hydroformylierungsreaktionen sowie ein Hydroformylierungsverfahren, bei dem die Verbindung als Ligand in einem Ligand-Metall-Komplex zur Umsetzung eines Olefins zu einem Aldehyd eingesetzt wird.

Erfindungsgemäße Komplexe umfassen ein Metallatom oder -ion und zumindest eine Monophosphitverbindung mit einer Struktur gemäß Formel **I** oder Formel **II** als Liganden. Das Metallatom oder -ion kann dabei insbesondere ausgewählt sein, aus der Gruppe, die Rhodium, Cobalt, Iridium und Ruthenium umfasst. Besonders bevorzugt ist das Metallatom oder-ion Rhodium. Optional kann der erfindungsgemäße Komplex auch zwei oder mehr Monophosphitverbindungen mit einer Struktur gemäß Formel **I** und/oder Formel **II** als Liganden aufweisen, wobei die Monophosphitverbindungen eine identische oder unterschiedliche Struktur aufweisen können.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Ein besonderer Vorteil erfindungsgemäßer Monophosphitverbindungen und erfindungsgemäßer Komplexe in der Hydroformylierung ist, dass diese auch bei der Umsetzung technischer Olefingemische zu Aldehyden als Liganden bzw. Katalysatoren verwendet werden können. Bevorzugt zeichnen sich erfindungsgemäße Monophosphitverbindungen und diese aufweisende Komplexe bei der Verwendung in Hydroformylierungsreaktionen darüber hinaus durch eine gute bis sehr gute Ausbeute aus.

Das Hydroformylierungsverfahren, bei dem erfindungsgemäße Monophosphitverbindungen als Ligand zur Umsetzung eines Olefins zu einem Aldehyd eingesetzt werden, umfasst die folgenden Verfahrensschritte:
a) Vorlegen eines Olefins,
b) Zugabe eines erfindungsgemäßen Komplexes oder alternativ einer erfindungsgemäßen Verbindung sowie einer ein Metallatom oder Metallion enthaltenden Verbindung, wobei das Metallatom oder Metallion vorzugsweise ausgewählt ist aus Rh, Ru, Co und Ir,
c) Zuführen von H₂ und CO,
d) Erwärmen des Reaktionsgemisches, wobei das Olefin zu einem Aldehyd umgesetzt wird.

Die Verfahrensschritte a) bis d) können dabei in beliebiger Reihenfolge erfolgen.

Die Reaktion wird bei üblichen Bedingungen durchgeführt, die dem Fachmann hinreichend bekannt sind.

Bevorzugt sind eine Temperatur von 80 °C bis 160 °C und ein Druck von 1 bis 300 bar. Besonders bevorzugt sind eine Temperatur von 100 °C bis 160 °C und ein Druck von 15 bis 250 bar.

In einer bevorzugten Ausführungsform ist das Metall Rh.

Mit dem erfindungsgemäßen Verfahren können unter Verwendung der erfindungsgemäßen Verbindungen α-Olefine, endständig verzweigte, innenständige und innenständig verzweigte Olefine hydroformyliert werden.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1-Buten, 2-Buten, 1-oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Di-n-buten, Di-iso-buten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische.

Die Erfindung wird nun genauer anhand von Ausführungsbeispielen beschrieben, die insbesondere die Herstellung erfindungsgemäßer Monophosphitverbindungen oder deren Zwischenstufen beinhalten.

Die Synthese relevanter Ethergruppen wird u.a. in "P. G. M. Wuts, T. W. Greene "Greenes's Protective Groups in Organic Synthesis", fourth edition, 2007, John Wiley and Sons; Hoboken, New Jersey" sowie in darin zitierten Literaturstellen behandelt. Weitere Literaturstellen zur Synthese ausgewählter Ethergruppen (in der vorliegenden Anmeldung Substituenten für W) werden im Folgenden gegeben:
- -Me (A.R. MacKenzie, C.J. Moody, C.W. Rees, Tetrahedron, 42, 3259, 1986),
- -CH₂OCH₃ (MOM) (F.R. van Heerden, J.J. van Zyl, G.J.H. Rall, E.V. Brandt, D.G. Roux, Tetrahedron Lett., 19, 661, 1978; W.R. Roush, D.S. Coffey, D.J. Madar, J. Am. Chem. Soc., 119, 11331, 1997; T.R. Kelly, C.T. Jagoe, Q. Li, J. Am. Chem. Soc., 111, 4522, 1989),
- -CH₂OCH₂C₆H₅ (BOM) (W. R. Roush, M.R. Michaelides, D.F. Tai, B.M. Lesur, W.K.M. Chong, D.J. Harris, J. Am. Chem. Soc. 111, 2984, 1989),
- -CH₂OCH₂CH₂OCH₃ (MEM) (E.J. Corey, R.L. Danheiser, S. Chandrasekaran, P. Siret, G.E. Kek, J.-L. Gras, J. Am. Chem. Soc., 100, 8031, 1978),
- -CH₂OCH₂CH₂Si(CH₃)₃ (SEM) (T. L. Shih, M.W. Wyvratt, H. Mrozik, J. Org. Chem., 52, 2029,1987),
- -CH₂SCH₃ (MTM) (R.A. Holton, R.G. Davis, Tetrahedron Lett., 18, 533, 1977),
- -CH₂SC₆H₅ (PTM) (R.A. Holton, R.V. Nelson, Synth. Commun., 10, 911, 1980),
- -CH₂CN (A. Benarab, S. Boye, L. Savelon, G. Guillaumet, Tetrahedron Lett., 34, 7567, 1993),
- -CF₂CHCl₂(H. Laatsch, Z. Naturforsch., B: Anorg. Chem., Org. Chem., 40b, 534,1985),
- -CH₂CH₂Cl (. Laatsch, Z. Naturforsch., B: Anorg. Chem., Org. Chem., 40b, 534, 1985),
- -CH₂CH₂Br (. Laatsch, Z. Naturforsch., B: Anorg. Chem., Org. Chem., 40b, 534,1985),
- -2-tetrahydropyranyl (H.N. Grant, V. Prelog, R.P.A. Sneeden, Helv. Chim. Acta., 46, 415, 1963),
- -CH(OC₂H₅)CH₃()J.H. Rigby, M.E. Mateo, J. Am. Chem. Soc., 199, 12655, 1997),
- -CH₂COC₆H₅ (J.B. Hendrickson, C. Kandall, Tetrahedron Lett., 11, 343, 1970),
- -CH₂COC₆H₄-4-Br (J.B. Hendrickson, C. Kandall, Tetrahedron Lett., 11, 343, 1970),
- -CH₂-*c*-C₃H₅ (W. Nagata, K. Okada, H. Itazaki, S. Uyeo, Chem. Pharm. Bull., 23, 2878, 1975),
- -CH₂CH=CH₂ (S.F. Martin, P.J. Garrison, J. Org. Chem., 47, 1513, 1982),
- -CH₂CH=C(CH₃)₂ (analog S.F. Martin, P.J. Garrison, J. Org. Chem., 47, 1513, 1982),
- -CH(CH₃)₂ (T. Sala, M.V. Sargent, J. Chem. Soc., Perkin Trans. I, 2593, 1979),
- -*c*-C₆H₁₁ (M. Engelhard, R.B. Merrifield, J. Am. CHem. Soc., 100, 3559, 1978),
- -C(CH₃)₃ (H.C. Beyerman, J.S.Bontekoe, Recl. Trav. Vhim. Pays-Bas, 81, 691, 1962),
- -CH₂C₆H₅ (M.C. Venuti, B.E. Loe, G.H. Jones, J.M. Young, J. Med. Chem., 31, 2132, 1988),
- -CH₂C₆H₃-2,4-(CH₃)₂ (R. Davis, J.M. Muchowski, Synthesis, 987, 1982),
- -CH₂C₆H₄-4-OCH₃ (J.D. White, J.C. Amedio, Jr., J. Org. Chem., 54, 736, 1989),
- -CH₂C₆H₄-*o*-NO₂ (B. Amit, E. Hazum, M. Fridkin, A. Patchornik, Int. J. Pept. Protein Res., 9, 91,1977),
- -CH₂C₄H₆-*p*-NO₂ (M.R. Pitts, J.R. Harrison, C.J. Moody, J. Chem. Soc., Perkin Trans. I, 955, 2001),
- -CH₂C₆H₃-2,6-Cl₂ (Y. Kiso, M. Satomi,. K Ukawa, T. Akita, J. Chem. Soc., Chem. Commun., 1063, 1980),
- -CH₂C₆H₃-3,4-Cl₂ (. Kiso, M. Satomi,. K Ukawa, T. Akita, J. Chem. Soc., Chem. Commun., 1063, 1980),
- -CH₂-9-anthyl (N. Kornblum, A. Scott, J. Am. Chem. Soc., 96, 590, 1974),
- -CH₂-4-pyridyl (A. Gosden, D. Stevenson, G.T. Young, J. Chem. Soc., Chem. Commun., 1123,1972),
- -Si(CH₃)₃ (Cl. Moreau, F. Roessac, J.M. Conia, Tetrahedron Lett. 11, 3527, 1970),
- -Si(CH₃)₂C(CH₃)₃ (R.C. Ronald, J.M. Lansinger, T.S. Lillie, C.J. Wheeler, J. Org. Chem., 44, 1421,1979),

### Allgemeine Arbeitsvorschriften:

Alle nachfolgend beschriebenen Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego, Christina Chai, Butterworth Heinemann (Elsevier), 6. Auflage, Oxford 2009). Alle präparativen Arbeiten erfolgten in ausgeheizten Gefäßen.

Die Charakterisierung der Produkte erfolgte mittels NMR-Spektroskopie. Chemische Verschiebungen (δ) werden in ppm angegeben. Die Referenzierung der 31P-NMR-Signale erfolgte gemäß: SR31 P = SR1 H * (BF31 P / BF1 H) = SR1 H * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Die Aufnahme von Kernresonanzspektren erfolgte mittels eines Bruker Avance 300 bzw. Bruker Avance 400, die gaschromatografische Analyse an Agilent GC 7890A.

### Einführung der Methyl-Gruppe:

### Beispiel 1: Darstellung von 3,3'-di-tert-butyl-2',5,5'-trimethoxy-[1,1'-biphenyl]-2-ol

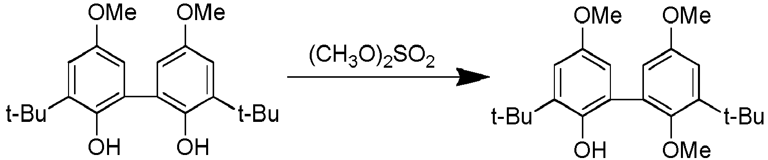

In einem sekurierten 1 L Schlenkkolben wurden 37.6 g (0,104 mol) 3,3'-Di-tert-butyl-5,5'-dimethoxy-[1,1'-biphenyl]-2,2'-diol und 17.3 g (0,124 mol) Kaliumcarbonat (wasserfrei) vorgelegt und unter Rühren in 10.0 ml (0,104 mol) Dimethylsulfat und 380 ml getrocknetem Aceton gelöst. Die erhaltene Suspension wurde 7,5 Stunden unter Rückfluss gekocht und anschließend auf Raumtemperatur abgekühlt.

Dann wurden 100 ml einer 2 molare Ammoniak-Lösung hinzugegeben und für 4 h bei Raumtemperatur gerührt. Anschließend wurde mit 200 ml Methylenchlorid ausgeschüttelt und die wässrige alkalische Phase mittels 1 molarer Salzsäure-Lösung (ca. 280 ml) sauer gestellt, die wiederum anschließend 3 mal 100 ml Methylenchlorid ausgeschüttelt wurde. Die erhaltenen organischen Phasen wurden vereinigt und mit 1 molarer Salzsäurelösung (100 ml) ausgeschüttelt. Die organische Phase wurde noch 3 mal mit VE-Wasser (200 ml) gewaschen und über Magnesiumsulfat getrocknet. Das Lösungsmittel wurde unter vermindertem Druck entfernt, das erhaltene Öl wurde mit 50 ml Isopropanol gewaschen, filtriert und anschließend getrocknet. Das Produkt konnte als weißer Feststoff in 30,8 g (Ausbeute 74,9 %) erhalten werden.

### Beispiel 2: Darstellung von 3,3',5,5'-tetra-tert.-butyl-2-metoxy-[1,1'-biphenyl]-2-ol

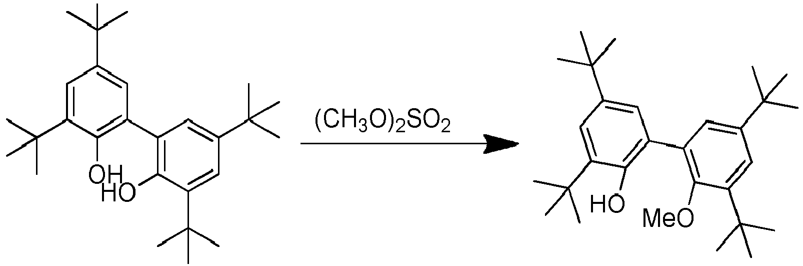

In einem sekurierten 1000 mL Schlenkkolben wurden 43 g (0,104 mol) 3,3',5,5'-Tetra-tert.-butyl-[1,1'-biphenyl]-2,2'-diol und 21,6 g (0,154 mol) Kaliumcarbonat vorgelegt und unter Rühren in 10,00 ml (0104 mol) Dimethylsulfat und 430ml getrocknetem Aceton gelöst. Die erhaltene Suspension wurde 6 Stunden unter Rückfluss gekocht und anschließend auf Raumtemperatur abgekühlt.

Nach Zugabe von 110 ml 2 molare Ammoniak-Lösung wurde 4 h gerührt. Anschließend wurde mit 200 ml Methylenchlorid ausgeschüttelt und die wässrige alkalische Phase mittels 1 molarer Salzsäure-Lösung (ca. 280 ml) sauer gestellt, die wiederum anschließend 3 mal 100 ml Methylenchlorid ausgeschüttelt wurde. Die erhaltenen organischen Phasen wurden vereinigt und mit 1 molarer Salzsäurelösung (100 ml) ausgeschüttelt.

Die organische Phase wurde noch 3 mal mit VE-Wasser (200 ml) gewaschen und über Magnesiumsulfat getrocknet. Das Lösungsmittel wurde unter vermindertem Druck entfernt, das erhaltene Öl wurde mit 50 ml Isopropanol gewaschen, filtriert und anschließend getrocknet. Das Produkt konnte als weißer Feststoff in 33,5 g (Ausbeute 75 %) erhalten werden.

### Einführen der Benzyl-Gruppe:

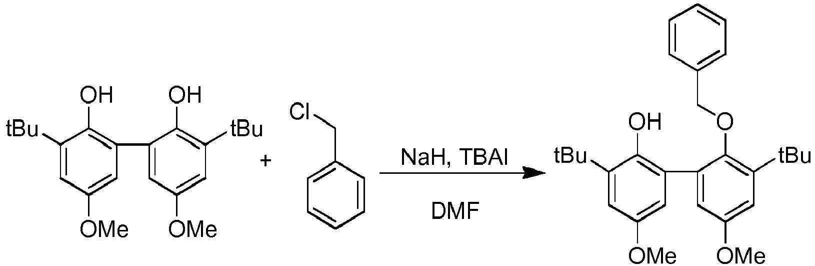

In einem sekurierten 500 mL Schlenkkolben wurden 35,8 g (100 mmol) 3,3'-di-tert-butyl-5,5'-dimethoxy-[1,1'-biphenyl]-2,2'-diol und 1,9 g (5 mmol) Tetra-n-butylammoniumiodid in 150 mL DMF suspendiert. Unter kräftigem Rühren wurden portionsweise 3 g (75 mmol) Natriumhydrid (60% in Mineralöl) zugegeben. Nach erfolgter Zugabe wurde für weitere 30 min bei Raumtemperatur nachgerührt und anschließend für 30 min auf 80 °C erwärmt. Unterdessen wurden in einem 100 mL Schlenkkolben 6,5 g (50 mmol) Benzylchlorid in 50 mL DMF gelöst und anschließend wurde diese Lösung langsam zur gekühlten Suspension zugetropft. Das Eisbad wurde entfernt und über Nacht gerührt.

Zur Reinigung wurde zweimal mit je 200 mL halbkonzentrierter Ammoniumchlorid-Lösung gewaschen, die organische Phase über Magnesiumsulfat getrocknet und im Rotationsverdampfer bei 80°C das Lösemittel entfernt, den Rückstand in 200 mL Toluol aufgeschlämmt und zweimal mit 200 mL 10%iger Natriumcarbonat-Lösung gewaschen. Anschließend wurde die organische Phase nochmals mit 200 mL Wasser gewaschen, über Magnesiumsulfat getrocknet und im Rotationsverdampfer einrotiert. Das Produkt konnte in 90 %iger Ausbeute erhalten werden (16,4g).

### Synthese der Chlorophosphite:

6-Chlorodibenzo[*d*,*f*][1,3,2]dioxaphosphepin wurde nach DE 10 2008 043 584 und 2-Chloro-4,4,5,5-tetraphenyl-1,3,2-dioxaphospholan nach DE 10 2006 058 682 hergestellt.

Die Herstellung von 2,2'-Bis-(3,5-ditert.-butyl)-phenol-chlorophosphit erfolgte nach folgender Vorschrift::

### Beispiel 3: Darstellung von 2,2'-Bis-(3,5-ditert.-butyl)-phenol-chlorophosphit

In einem sekurierten 500 mL Schlenkkolben wurden 41g (0,1 mol) 2,2'-Bis-(3,5-ditert.-butyl)-phenol und 30,7 g (42,3 mL; 0,3 mol) getrocknetes Triethylamin in 300 mL getrocknetem Toluol gelöst.
In einem zweiten sekurierten Schlenkkolben (1000 mL) löste man 13,9 g (8,8 mL; 0,1 mol) Phosphortrichlorid in 600 mL getrocknetem Toluol und tropfte unter kräftigem Rühren zu dieser Lösung langsam und stetig bei einer Temperatur zwischen -5 und 0 °C die zuvor hergestellte Diphenol-Triethylamin-Toluol-Lösung hinzu. Die Lösung ließ man über Nacht auf Raumtemperatur erwärmen. Das entstehende Ammoniumchlorid wurde abfiltriert und das Lösemittel unter vermindertem Druck bis zur Trockene eingeengt. Das Produkt konnte in 98%iger Ausbeute erhalten werden (47g).

Alle anderen Chlorophosphite lassen sich in analoger Weise, d.h. durch Zusatz von Phosphortrichlorid in Gegenwart einer Base, herstellen. Siehe hierzu auch "Phosphorous(III) Ligands in Homogeneous Catalysis - Design and Synthesis" von Paul C.J. Kamer und Piet W.N.M. van Leeuwen; John Wiley and Sons, 2012; unter anderem S. 94 ff. und darin zitierte Literaturstellen.

### Beispiel 4: Darstellung von Biphenyl-3,3',5,5'-Tetratert.-butyl-2-hydroxy-2'-dichlorophosphit

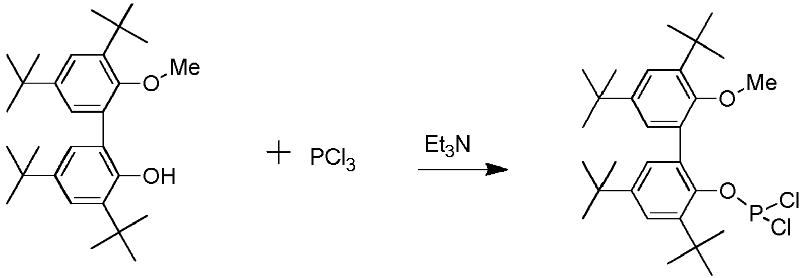

In einem sekurierten 250 ml Schlenkkolben wurde 10,62 g (0,025 mol) 3,3',5,5'-Tetratert.-butyl-2-hydroxy-2'-methoxy-biphenyl unter Rühren in 50 ml getrocknetem Toluol gelöst und mit 3,5 ml (0,025 mol) Triethylamin versetzt. Zu der erhaltenen Lösung tropft man bei Raumtemperatur unter kräftigem Rühren 2,2 ml (0,025 mol) Phosphortrichlorid hinzu und erhitzt dann für 4 Stunden auf 105 °C. Zur Aufarbeitung filtriert man das ausgefallene Ammoniumchlorid ab und wäscht 2 mal mit 25 ml Toluol nach. Das Filtrat wird bis zur Trockne eingeengt. Das Produkt konnte in 63%iger Ausbeute erhalten werden.

### Beispiel 5: Darstellung von Dichloro((3,3'-di-tert-butyl-2',5,5'-trimethoxy-[1,1'-biphenyl]-2-yl)oxy)phosphine

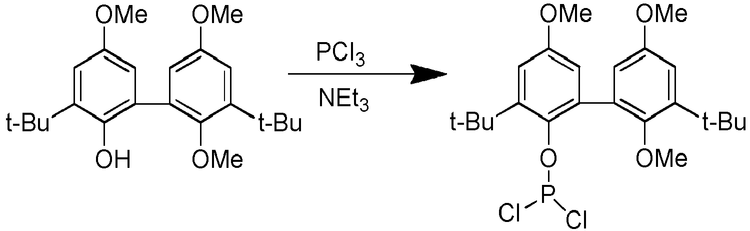

In einem sekurierten 250 ml Schlenkkolben wurden 28,4 g (0,072 mol) 3,3'-Di-tert-butyl-2',5,5'-trimethoxy-[1,1'-biphenyl]-2-ol unter Rühren in 200 ml getrocknetem Toluol 40.0 ml (0,282 mol) entgastes Triethylamin gelöst.

In einem zweiten 500 ml Schlenkkolben wird zunächst 200 ml getrocknetes Toluol vorgelegt und dann 25 ml (0,281 mol) Phosphortrichlorid zugegeben. Nun wird unter kräftigem Rühren die zuvor hergestellte Phenol/Amin/Toluol-Lösung innerhalb von 1 h bei Raumtemperatur zu der Phosphortrichlorid/Toluol-Lösung zugetropft. Nach vollständiger Zugabe wird für 4 h auf 80 °C erwärmt und über Nacht auf Raumtemperatur abgekühlt.

Die Reaktionsmischung wird filtriert, dreimal mit 50 ml getrocknetem Toluol gewaschen und das Filtrat wird bis zur Trockne eingeengt. Das Produkt konnte in 83%iger Ausbeute erhalten werden (35,3 g).

### Synthese der Monophosphite:

### Beispiel 6: Darstellung von 6-((3,3',5,5'-Tetra-tert-butyl-2'-methoxy-[1,1'-biphenyl]-2-yl)oxy)dibenzo[d,f][1,3,2]dioxaphosphepine

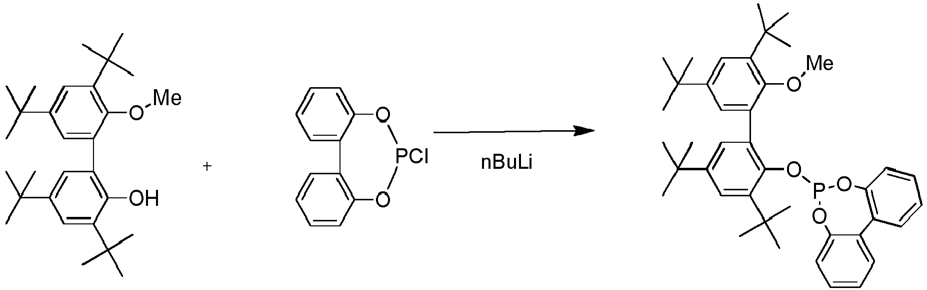

In einem sekurierten 250 ml Schlenkkolben wurden 5 g (0,012 mol) 3,3',5,5'-Tetratert.-butyl-2-hydroxy-2'-methoxy-biphenyl in 50 ml getrocknetem THF gelöst. Dann wurden 7,5 ml Butyllithium (1,6 molare Lösung, 0,012 mol) unter kräftigem Rühren zu der Tetrahydrofuran/Phenol - Mischung bei -20 °C getropft. Nach vollständiger Zugabe wurde eine Stunde bei 0 °C gerührt. In einem zweiten sekurierten Schlenk (100 ml) wurden 3 g (0,012 mol) 6-Chlorodibenzo[*d*,*f*][1,3,2]dioxaphosphepin unter Rühren in 50 mL getrocknetem THF aufgelöst. Anschließend wurde die Chlorophosphit-Lösung zu der vorbereiteten Phenol-Lösung unter kräftigem Rühren bei 0 °C getropft. Das Reaktionsgemisch wurde für 2 Stunden bei 60 °C erwärmt. Nach dem Abkühlen auf Raumtemperatur wurde das Lösungsmittel unter vermindertem Druck entfernt.

Der erhaltene Rückstand wurde in 50 ml Methylenchlorid aufgenommen, filtriert um das Lithiumchlorid abzutrennen, und dann wieder bis zur Trockene eingeengt und mit Isopropanol gewaschen. Das Produkt konnte in 5,5 g (72,8%) erhalten werden.

### Beispiel 7: Darstellung von 2,4,8,10-Tetra-tert-butyl-6-((3,3',5,5'-tetra-tert-butyl-2'-methoxy-[1,1'-biphenyl]-2-yl)oxy)dibenzo[d,f][1,3,2]dioxaphosphepine

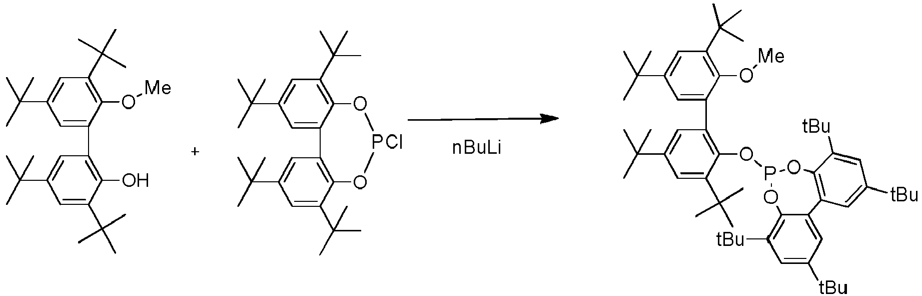

In einem sekurierten 250 ml Schlenkkolben wurden 5 g (0,012 mol) 3,3',5,5'-Tetratert.-butyl-2-hydroxy-2'-methoxy-biphenyl in 50 ml getrocknetem THF aufgelöst und bei -20 °C mit 7,5 ml Butyllithium (1,6 molare Lösung, 0,012 mol) versetzt. Nach vollständiger Zugabe wurde noch eine Stunde bei 0 °C gerührt. In einem zweiten sekurierten Schlenkkolben (100 ml) wurden 5,7 g (0,012 mol) 2,2'-Bis-(3,5-ditert.-butyl)-phenol-chlorophosphit unter Rühren in 50 mL getrocknetem THF aufgelöst. Dann wurde unter kräftigem Rühren die Chlorophosphit-Lösung zu der vorbereiteten Phenol-Lösung bei 0 °C getropft. Nach vollständiger Zugabe wurde die Reaktionsmischung über Nacht langsam auf Raumtemperatur erwärmt. Dann wurde die Reaktionsmischung für 20 Stunden auf 60 °C erwärmt. Zur Aufarbeitung wurde das Lösungsmittel bei Raumtemperatur unter vermindertem Druck entfernt. Der erhaltene Rückstand wurde in 50 ml Methylenchlorid aufgenommen und das zurückbleibende Lithiumchlorid wurde abfiltriert. Das erhaltene Filtrat wurde mit ca. 40 ml getrocknetem Acetonitril gewaschen und erneut abfiltriert. Der Rückstand wurde mit ca. 50 ml Isopropanol und ca. 50 ml Pentan gewaschen. Das Produkt konnte in 35 %iger Ausbeute erhalten werden.

### Beispiel 8: Darstellung von Bis(2,4-di-tert-butylphenyl)-(3,3',5,5'-tetra-tert-butyl-2'-methoxy-[1,1'-biphenyl]-2-yl)phosphit

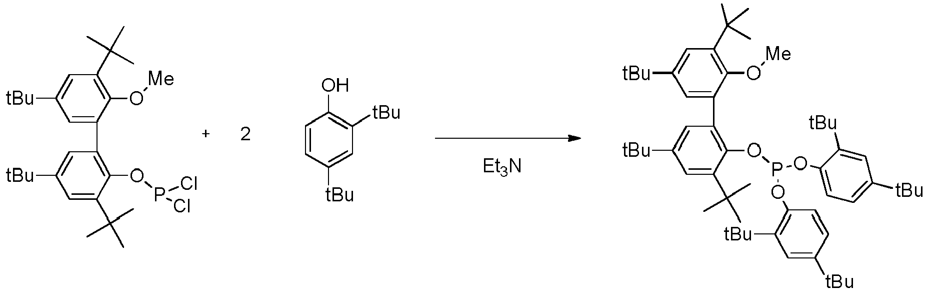

In einem sekurierten 250 ml Schlenk wurden 2,6 g (0,012 mol) 2,4-Di-tert.-butylphenol unter Rühren in 100 ml getrocknetem Toluol gelöst und mit 3,5 ml (0,025 mol) getrocknetem Triethylamin versetzt. In einem zweiten sekurierten Schlenk (100 ml) wurden 3,3 g (0,006 mol) Biphenyl-3,3',5,5'-Tetratert.-butyl-2-methoxy-2'-dichlorophosphit unter Rühren in 100 ml getrocknetem Toluol gelöst. Anschließend tropfte man die Chlorophosphit/Toluol-Lösung unter kräftigem Rühren bei 0 - (-4) °C in die Phenol/Amin/Toluol-Lösung und ließ die Lösung über Nacht auf Raumtemperstur erwärmen. Das Ammoniumchlorid absetzen gelassen und zur Umsatzprüfung von der obenstehenden Lösung eine Probe für das GC/MS entnommen. Danach wurde das entstandene Ammoniumchlorid abfiltriert und das Lösungsmittel unter vermindertem Druck bis zur Trockenen eingeengt. Das enstandene Öl wurde aus getrocknetem Acetonitril umkristallisiert und getrocknet. Das Produkt konnte in 52%iger Ausbeute erhalten werden.

### Beispiel 9: Darstellung von 6-((3,3'-Di-tert-butyl-2,5,5'-trimethoxy-[1,1'-biphenyl]-2-yl)oxy)-2,4,8,10-tetramethyldibenzo[d,f][1,3,2]dioxaphosphepine

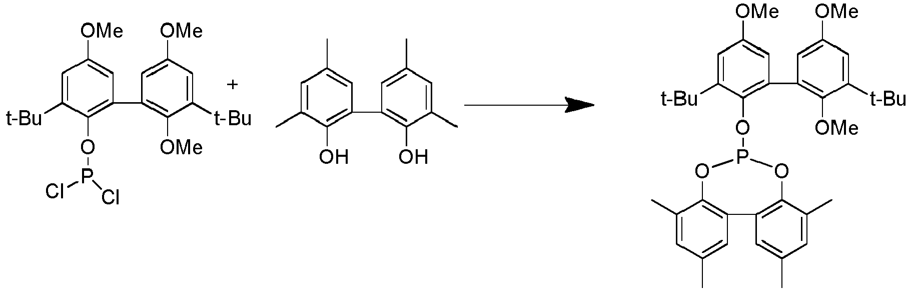

In einem sekurierten 500 ml Schlenkkolben wurden 4.1 g (0,008mol) Dichloro((3,3'-di-tert-butyl-2',5,5'-trimethoxy-[1,1'-biphenyl]-2-yl)oxy)phosphine in 100 ml entgastem Acetonitril gelöst.
In einem zweiten sekurierten Schlenkkolben (500 ml) wurden 1.89 g (0,008 mol) 3,3',5,5'-tetramethyl-(1,1'-biphenyl)-2,2'-diol in 100 ml entgastem Acetonitril und 2.3 ml (0,016 mol) N,N'-Dimethylaminobutan gelöst. Anschließend wird zur Chlorophosphit-Lösung langsam und stetig bei Raumtemperatur in die "Biphenol" - Amin Lösung getropft und 2,5 h bei Raumtemperatur gerührt.

Das Lösemittel wurde bei 40 °C unter vermindertem Druck entfernt, der erhaltene Rückstand wurde in Toluol aufgenommen, das Hydrochlorid abfiltriert und das Lösungsmittel erneut unter vermindertem Druck entfernt. Das Produkt konnte in 72%iger Ausbeute (4.1 g) erhalten werden.

### Beispiel 10: Darstellung von 2,4,8,10-Tetramethyl-6-((3,3',5,5'-tetra-tert-butyl-2'-methoxy-[1,1'-biphenyl]-2-yl)oxy)dibenzo[d,f][1,3,2]dioxaphosphepine

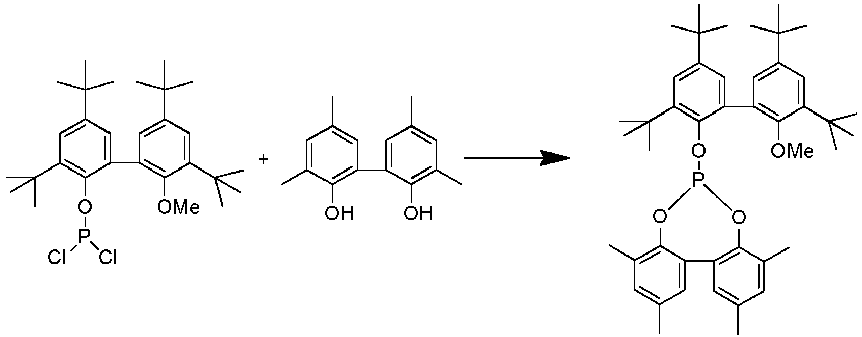

In einem sekurierten 250 ml Schlenkkolben wurden 10 g (0,017 mol) Biphenyl-3,3',5,5'-Tetratert.-butyl-2-methoxy-2'-dichlorophosphit in 50 ml entgastem Toluol gelöst. In einem zweiten Schlenkkolben (100 ml) wurden 5 g (0,019mol) 3,3',5,5'-Tetramethyl-[1,1'-biphenyl]-2,2'-diol in 50 ml entgastem Toluol und 4ml (0,029mol) entgastem Triethylamin gelöst. Anschließend wurde die Phenol/Amin/Toluol-Lösung tropfenweise zu der Chlorophosphit/Toluol-Lösung unter kräftigem Rühren und bei Raumtemperatur zugegeben.

Nach vollständiger Zugabe wurde die Reaktionsmischung für eine Stunde auf 80 °C erwärmt und über Nacht im Ölbad wieder auf Raumtemperatur abgekühlt.

Zur Aufarbeitung wurde das entstandene Aminhydrochlorid abfiltriert und die erhaltene Mutterlauge unter vermindertem Druck bis zur Trockne eingeengt. Anschließend wurde der Feststoff (8,7 g) nochmals in 30ml Toluol aufgelöst. In einem anderen Schlenkkolben wurden 260 ml Methanol, 13ml entgastes Wasser und 13ml entgastes Dimethylaminobutan gefüllt. Unter Rühren wurde dann langsam die Monophosphit/Toluol-Lösung bei Raumtemperatur zu der Methanol-Lösung getropft und eine Stunde bei Raumtemperatur gerührt, dann mittels Eisbad auf 0 °C abgekühlt und nochmal zwei Stunden gerührt. Die entstandene Suspension wurde abfiltriert. Das Produkt konnte als Feststoff erhalten werden (6,4 g).

### Durchführung der Katalyseversuche:

Um die Katalyseeigenschaften der erfindungsgemäßen Verbindungen bzw. Komplexe zu testen, wurden diese gemäß der folgenden allgemeinen Versuchsbeschreibung in der Hydroformylierung verschiedener Olefine eingesetzt.

### Allgemeine Versuchsbeschreibung:

In einem 100 ml-Autoklaven der Fa. Parr Instruments wurden bei verschiedenen Temperaturen sowie bei jeweils 20 und 50 bar Synthesegasdruck (CO/H₂ = 1:1 (Vol-%)) verschiedene Olefine hydroformyliert. Als Precursor wurden bei einer Katalysatorkonzentration von 40 ppm Rh bezogen auf das gesamte Reaktionsgemisch 0.005 g Rh(acac)(CO)₂ vorgelegt, bei einer Konzentration von 100 ppm Rh entsprechend 0.0123 g Rh(acac)(CO)₂. Als Lösemittel wurden jeweils 40 bis 46 g Toluol verwendet.

Die zu testenden Verbindungen wurde in unterschiedlichen molaren Überschüssen relativ zum Rhodium verwendet. Zusätzlich wurden als GC-Standard ca. 0.5 g Tetraisopropylbenzol (TIPB) zugefügt. Ca. 6 g Edukt wurden nach Erreichen der vorgesehenen Reaktionstemperatur zudosiert.

Während der Reaktion wurde der Druck über eine Synthesegasdosierung mit Massendurchflussmesser und Druckregler konstant gehalten. Die Rührerdrehzahl betrug 1200 min⁻¹. Nach jeweils 3 bzw. 12 Stunden wurden Proben aus der Reaktionsmischung gezogen.

### Beispiel 11: Katalyseversuche mit den Verbindungen L1, L2 und L3

Die Verbindungen **L1, L2** und **L3,** deren Strukturen nachfolgend angegeben sind, wurden gemäß der vorstehenden allgemeinen Versuchsbeschreibung auf ihre Eignung zur Katalyse von Hydroformylierungsreaktionen getestet.

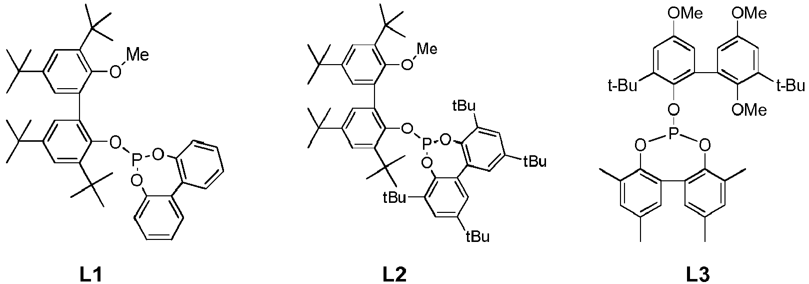

Als Vergleichsverbindungen wurden die nicht erfindungsgemäßen Verbindungen **L4** und **L5** verwendet, deren Synthese im Nachfolgenden erläutert wird.

Bei den Verbindungen **L4** und **L5** handelt es sich ebenfalls um Monophosphitverbindungen, die jedoch anstelle der Me-Gruppe eine BOC-Gruppe tragen (tert.-Butyloxycarbonyl = BOC).

### Synthesevorschrift der Vergleichsverbindungen:

### Vorstufen:

### Einführung der BOC-Gruppe:

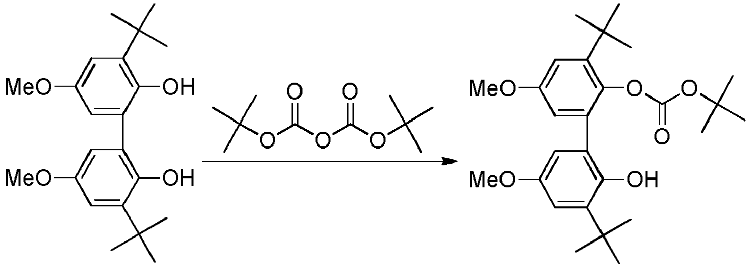

In einem 2L Schlenkkolben werden 400 mmol (143,8 g) 3,3'-Di-tert.-butyl-5,5'-dimethoxy-[1,1'-biphenyl]-2,2'diol und 40 mmol (4,8 g) N,N-Dimethylaminopyridin (DMAP) in 900 mL CH₂Cl₂ gelöst. Anschließend wurden bei Raumtemperatur 400 mmol (88 g) Di-tert-butyldicarbonat in 280 ml CH₂Cl₂ gelöst, in einen 500 ml Tropftrichter überführt und innerhalb einer Stunde bei 32 °C zu der Biphenol/DMAP Lösung getropft. Die Lösung wurde über Nacht bei Raumtemperatur gerührt. Am nächsten Morgen wurde das Lösungsmittel unter vermindertem Druck entfernt. Der leicht wachsartige, rötliche Rückstand wurde mit 800 ml n-Heptan versetzt und über Nacht gerührt. Dabei erhielt man einen weißen Rückstand, der abfiltriert, zweimal mit 50 ml n-Heptan nachgewaschen und dann getrocknet wurde. Das Zielprodukt konnte als weißer Feststoff (161,6 g, 84%) erhalten werden. ¹H-NMR (Tolul-d₈): 95% und weitere Verunreinigungen.

### Umsetzung von tert-Butyl-(3.3'-di-tert-butyl-2'-hydroxy-5,5'-dimethoxy-[1,1'-biphenyl]1-2-yl)-carbonat mit Phosphortrichlorid:

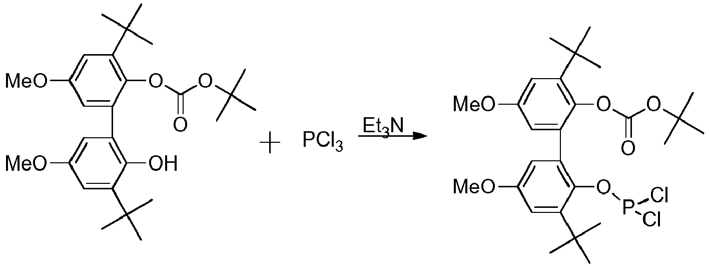

In einem sekurierten 250 ml Schlenkkolben wurden 12 g (0,026 mol) tert-Butyl-(3,3'-di-tert-butyl-2'-hydroxy-5,5'-dimethoxy-[1,1'-biphenyl]-2-yl)-carbonat unter Rühren in 120 ml getrocknetem Toluol und 12,8 ml (0,091 mol) Triethylamin gelöst.

In einem zweiten 500 ml Schlenkkolben wurden zunächst 100 ml getrocknetes Toluol mit 8,1 ml (0,091 mol) Phosphortrichlorid verrührt. Anschließend wurde die Phosphortrichlorid-Toluol-Lösung zu der zuvor hergestellten Carbonat-Amin-Toluol-Lösung innerhalb von 30 Minuten bei Raumtemperatur zugetropft. Nach vollständiger Zugabe wurde für 30 Minuten auf 80 °C erwärmt und über Nacht auf Raumtemperatur abgekühlt.

Am nächsten Morgen wurde die Mischung filtriert, mit 50 ml getrocknetem Toluol nachgewaschen und das Filtrat bis zur Trockne eingeengt. Das Zielprodukt konnte als Feststoff (13,1 g, 89%) erhalten werden. ³¹P-NMR (202,4 MHz, Toluol-d₈): 203,2 und 203,3 ppm (100%).

### Synthese von L4:

### Umsetzung von tert-Butyl-(3,3'-di-tert-butyl-2'-((dichlorophosphino)oxy)-5,5'-dimethoxy-[1,1'-biphenyl]-2-yl)-carbonat mit 3,3,5,5-Tetra-tert.-butyl-biphenol

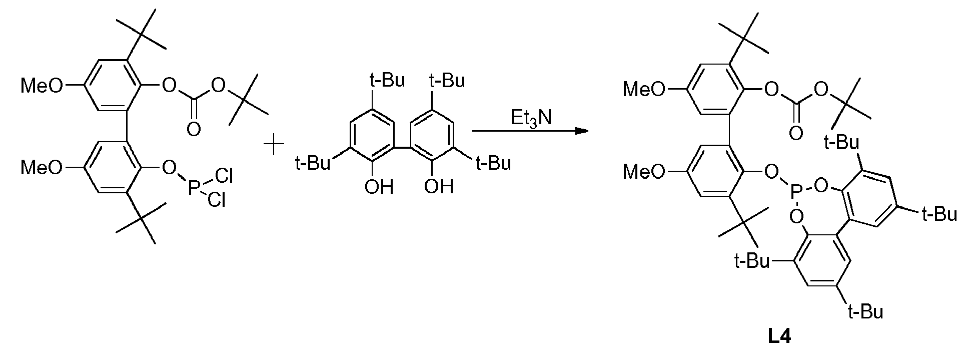

In einem sekurierten 250 ml Schlenkkolben wurden 7.0 g (0,0125 mol) tert-Butyl-(3,3'-di-tert-butyl-2'-((dichlorophosphino)oxy)-5,5'-dimethoxy-[1,1'-biphenyl]-2-yl)-carbonat in 100 ml getrocknetem Acetonitril gelöst.

In einem zweiten sekurierten Schlenkkolben (100 ml) wurden 5.1 g (0,0125 mol) 3,3',5,5'-Tetra-tert.-butyl-biphenol in 60 ml getrocknetem Acetonitril und 4.2 ml (0,03 mol) getrocknetem Triethylamin unter Rühren aufgelöst. Im Anschluss wurde die Biphenol-Triethylamin-Lösung langsam bei Raumtemperatur zu der Chlorophosphit-Lösung getropft und über Nacht gerührt. Ein Teil des Lösungsmittels wurde unter vermindertem Druck entfernt. Der ausgefallene Feststoff wurde filtriert und getrocknet. Das Zielprodukt konnte als weißer Feststoff (10,2 g, 91%) erhalten werden. 31P-NMR (202,4 MHz, toluene-d8): 142,7 ppm (100%).

### Synthese von L5:

### Umsetzung von tert-Butyl-(3,3'-di-tert-butyl-2'-((dichlorophosphino)oxy)-5,5'-dimethoxy-[1,1'-biphenyl]-2-yl)-carbonat mit 2,2'-Biphenol

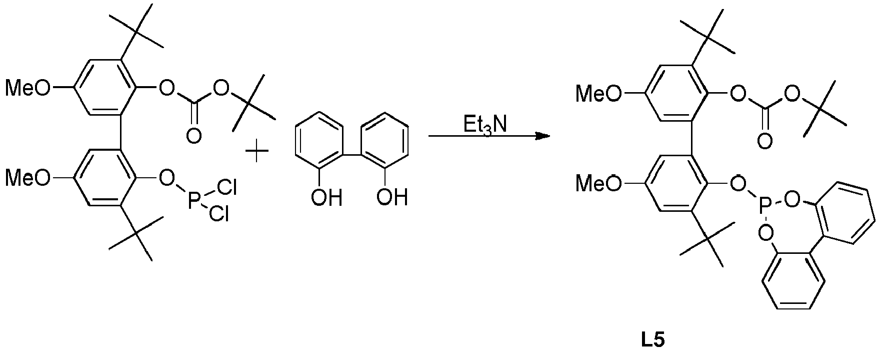

In einem sekurierten 250 ml Schlenkkolben wurden 10,5 g (0,019 mol) tert-Butyl-(3,3'-di-tert-butyl-2'-((dichlorophosphino)oxy)-5,5'-dimethoxy-[1,1'-biphenyl]-2-yl)-carbonat unter Rühren in 50 ml entgastem Acetonitril gelöst.

In einem zweiten sekurierten Schlenkkolben (250 ml) wurden 3,6 g (0,019 mol) 2,2'-Biphenol in 40 ml entgastem Acetonitril und 6,3 ml (0,045 mol) getrocknetem Triethylamin unter Rühren aufgelöst. Anschließend wurde zu der Biphenol-Triethylamin-Lösung langsam bei Raumtemperatur die Chlorophosphit-Mischung hinzugetropft und über Nacht bei Raumtemperatur gerührt. Der erhaltene Feststoff wurde filtriert und getrocknet. Das Zielprodukt konnte als weißer Feststoff (11,5 g, 90%) erhalten werden. ³¹P-NMR (202,4 MHz, Toluol-d₈):146,2 ppm (100%).

Die erfindungsgemäßen Monophosphitverbindungen **L1** bis **L3** sowie die Vergleichsverbindungen **L4** und **L5** wurden jeweils zur Katalyse von Hydroformylierungsreaktionen eingesetzt. Dabei wurde als Olefin jeweils Di-n-Buten (eine Mischung aus Isomeren von n-Octenen (ca. 16 %), 3-Methyl-heptenen (ca. 65 %) und 3,4-Dimethylhexenen (ca. 19 %)) eingesetzt.

Die Reaktionsparameter und die jeweiligen erzielten Ausbeuten sind in der nachstehenden Tabelle 1 zusammengefasst:

**Tabelle 1: Übersicht über die Reaktionsparameter**

| Verbindung | Synthesegasdruck in [bar] | T in [°C] | c(Rh) in ppm | P:Rh | Ausbeute in % |
|---|---|---|---|---|---|
| L1* | 50 | 130 | 100 | 20 | 92 |
| L1* | 50 | 140 | 100 | 20 | 84 |
| L2* | 50 | 130 | 100 | 20 | 79 |
| L3* | 50 | 120 | 100 | 20 | 90 |
| L3* | 50 | 130 | 100 | 20 | 89 |
| L4 | 50 | 140 | 100 | 20 | 38 |
| L5 | 50 | 140 | 100 | 10 | 74 |

| | | | | | |
|---|---|---|---|---|---|
| * erfindungsgemäße Verbindung | | | | | |

Wie Tabelle 1 zu entnehmen ist, wurden die erfindungsgemäßen Verbindungen **L1, L2** und **L3** sowie die Vergleichsverbindungen **L4** und **L5** zur Katalyse in Hydroformylierungsreaktionen bei jeweils konstanter Temperatur im Bereich von 120 °C bis 140 °C eingesetzt.

Mit allen verwendeten erfindungsgemäßen Verbindungen wurde eine gute bis sehr gute Ausbeute erzielt, die jeweils oberhalb der mit der Vergleichsverbindung **L5** erzielten Ausbeute und deutlich oberhalb der mit der Vergleichsverbindung **L4** erzielten Ausbeute lag.

Die erfindungsgemäßen Verbindungen zeichnen sich daher durch eine sehr gute Katalyseeignung aus und sind somit als Katalysatoren für die Hydroformylierungsreaktion von technischen Olefingemischen sehr gut geeignet.

## Patentansprüche

1. Verbindung, welche eine der beiden allgemeinen Strukturen **I** oder **II** aufweist: 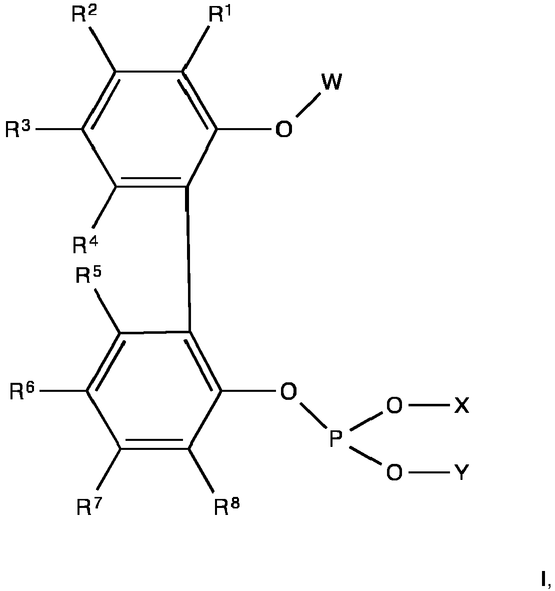 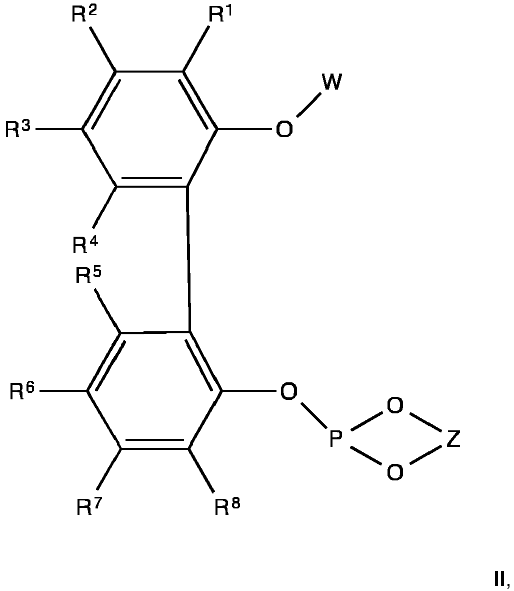
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM),-CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-C-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-N0₂, - CH₂C₄H₆-*p*-NO₂, -CH₂CeH₃-2,6-Cl₂, -CH₂C₆H₃-3,4-Cl₂, -CH₂-9-anthyl, -CH₂-4-pyridyl, Si(CH₃)₃, -Si(CH₃)₂C(CH₃)₃;
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, (C₁-C₁₂)-Alkyl-(C₆-C₂₀)-Aryl, -(C₆-C₂₀)-Aryl-COO-(C₁-C₁₂)-Alkyl, -(C₆-C₂₀)-Aryl-CONH-(C₁-C₁₂)-Alkyl, -(C₆-C₂₀)-Aryl-CON[(C₁-C₁₂)-Alkyl]₂, -(C₄-C₂₀)-Heteroaryl, -(C₄-C₂₀)-Heteroaryl-(C₁-C₁₂)-Alkyl, -(C₅-C₈)-Cycloalkyl, -(C₅-C₈)-Heterocycloalkyl, -(C₆-C₂₀)-Aryl-CO-(C₆-C₂₀)-Aryl;
wobei **Z** ausgewählt ist aus: -(C₁-C₁₂)-AlkYl-, -(C₆-C₂₀)-Aryl-, -(C₆-C₂₀)-Aryl-(C₁-C₁₂)-Alkyl-, -(C₁-C₁₂-Alkyl-(C₆-C₂₀-Aryl-, -(C₄-C₂₀)-Heteroaryl-, -(C₆-C₂₀)-Aryl-CO-(C₆-C₂₀)-Aryl-, -(C₆-C₂₀)-Aryl-(C₆-C₂₀)-Aryl-.
und wobei die genannten Alkyl-, Heteroalkyl-, Cycloalkyl-, Heterocycloalkyl-, Aryl- und Heteroarylgruppen substituiert sein können.

2. Verbindung nach Anspruch **1,**
wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₁-C₁₂)-Alkyl-(C₆-C₂₀)-Aryl, -(C₄-C₂₀)-Heteroaryl und -(C₅-C₈)-Cycloalkyl.

3. Verbindung nach einem der Ansprüche 1 oder 2,
wobei Z ausgewählt ist aus: -(C₁-C₁₂)-Alkyl-, -(C₆-C₂₀)-Aryl-, -(C₆-C₂₀)-Aryl-(C₁-C₁₂)-Alkyl-, -(C₁-C₁₂)-Alkyl-(C₆-C₂₀)-Aryl- und - (C₆-C₂₀)-Aryl-(C₆-C₂₀)-Aryl-.

4. Verbindung nach einem der Ansprüche 1 bis 3,
wobei W ausgewählt ist aus: -(C₁-C₁₂)-Alkyl, -Me, -CH₂CH₃, -CH₂CH₂CH₃, -CH₂-C-C₃H₅, -CH(CH₃)₂, -*c*-C₆H₁₁, -C(CH₃)₃, - CH₂C₆H₅, -CH₂C₆H₃-2,4-(CH₃)₂, -CH₂C₆H₄-4-OCH₃,-CH₂-9-anthyl, -CH₂-4-pyridyl.

5. Verbindung nach Anspruch 1, welche die allgemeine Struktur III aufweist: 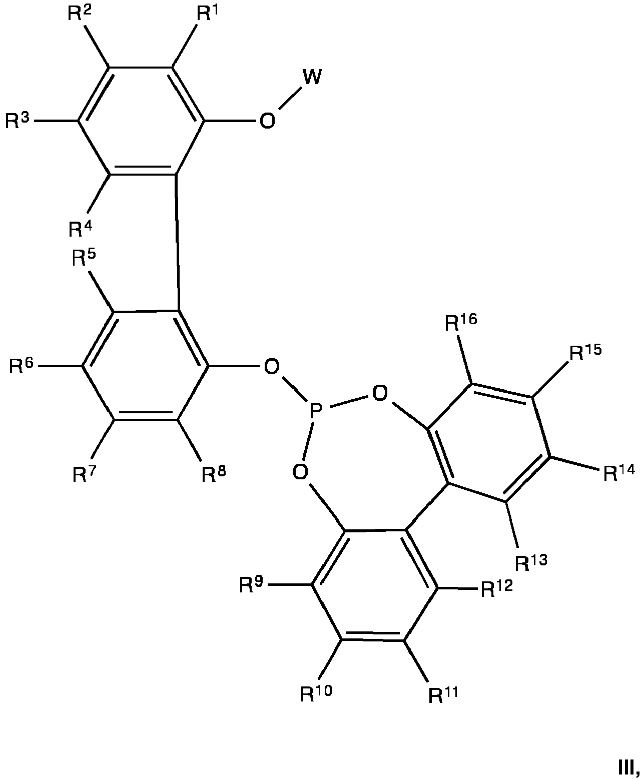
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM),-CH₂CN, -CF₂CHCl₂, -CH₂CH₂Cl, -CH₂CH₂Br, -2-tetrahydropyranyl, -CH(OC₂H₅)CH₃, - CH₂COC₆H₅, -CH₂COC₆H₄-4-Br, -CH₂-*c*-C₃H₅, -CH₂CH=CH₂, -CH₂CH=C(CH₃)₂, -CH(CH₃)₂, -*c-*C₆H₁₁, -C(CH₃)₃, -CH₂C₆H₅, -CH₂C₆H₃-2,4-(CH₃)₂, -CH₂C₆H₄-4-OCH₃, -CH₂C₆H₄-O-NO₂, - CH₂C₄H₆-*p*-NO₂, -CH₂C₆H₃-2,6-Cl₂, -CH₂C₆H₃-3,4-Cl₂, -CH₂-9-anthyl, -CH₂-4-pyridyl, -Si(CH₃)₃, - Si(CH₃)₂C(CH₃)₃;
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
und wobei R⁹, R¹⁰, R¹¹, R¹², R¹³, R¹⁴, R¹⁵, R¹⁶ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

6. Verbindung nach einem der Ansprüche 1 bis 5, wobei die Verbindung eine Struktur aufweist, die ausgewählt ist unter **L1, L2** und **L3:**

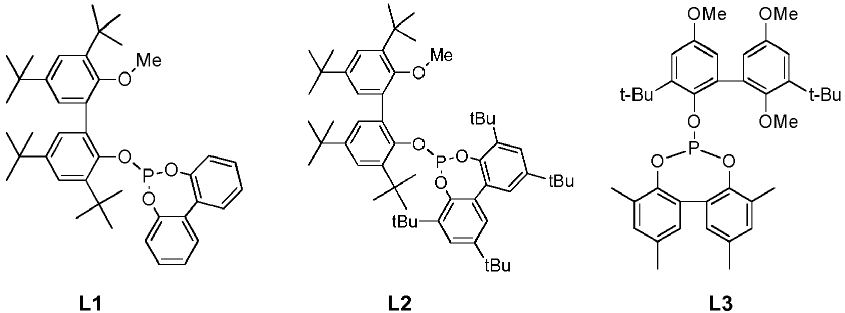

7. Verfahren zur Herstellung einer Verbindung, welche eine der beiden allgemeinen Strukturen I oder **II** aufweist: 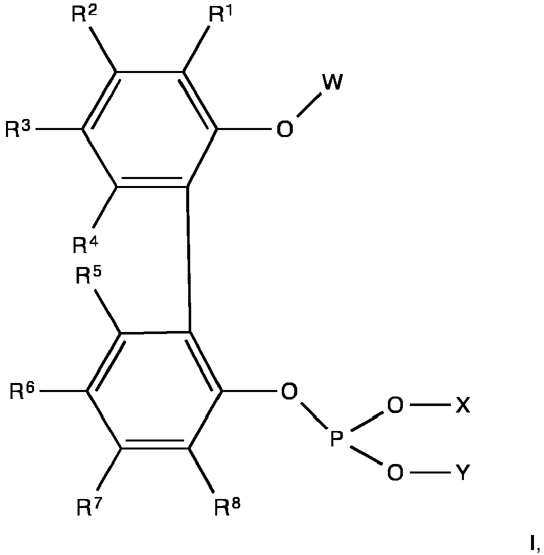 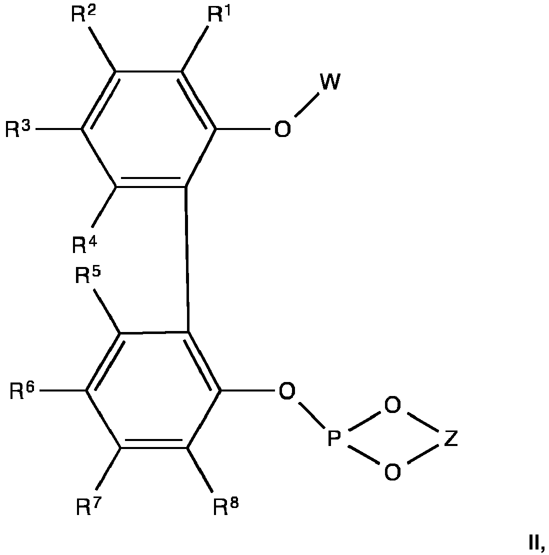
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM),-CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-C-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, - CH₂C₄H₆-*p*-NO₂, -CH₂CeH₃-2,6-Cl₂, -CH₂C₆H₃-3,4-Cl₂, -CH₂-9-anthyl, -CH₂-4-pyridyl, Si(CH₃)₃, -Si(CH₃)₂C(CH₃)₃;
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, - (C₁-C₁₂)-Alkyl-(C₆-C₂₀)-Aryl, -(C₆-C₂₀)-Aryl-COO-(C₁-C₁₂)-Alkyl, -(C₆-C₂₀)-Aryl-CONH-(C₁-C₁₂)-Alkyl, -(C₆-C₂₀)-Aryl-CON[(C₁-C₁₂)-Alkyl]₂, -(C₄-C₂₀)-Heteroaryl, -(C₄-C₂₀)-Heteroaryl-(C₁-C₁₂)-Alkyl, -(C₅-C₈)-Cycloalkyl, -(C₅-C₈)-Heterocycloalkyl, -(C₆-C₂₀)-Aryl-CO-(C₆-C₂₀)-Aryl;
und wobei Z ausgewählt ist aus: -(C₁-C₁₂)-Alkyl-, -(C₆-C₂₀)-Aryl-, -(C₆-C₂₀)-Aryl-(C₁-C₁₂)-Alkyl-, -(C₁-C₁₂-Alkyl-(C₆-C₂₀-Aryl-, -(C₄-C₂₀)-Heteroaryl-, -(C₆-C₂₀)-Aryl-CO-(C₆-C₂₀)-Aryl-, -(C₆-C₂₀)-Aryl-(C₆-C₂₀)-Aryl-;
wobei das Verfahren die folgenden Schritte umfasst:
a) Vorlegen eines Eduktes,
b) Zugabe eines Alkylierungsreagenzes,
c) Zugabe einer Verbindung, die Phosphor und Chlor enthält,
d) Erhalten eines Produktes.

8. Verfahren nach Anspruch 7, wobei ein Zwischenprodukt mit einer Struktur gemäß Formel **40** gebildet wird 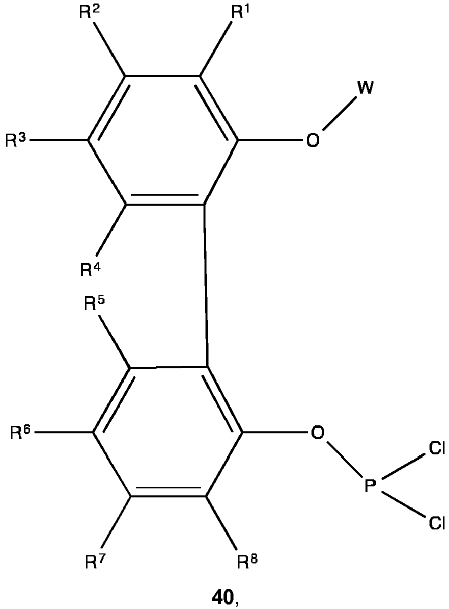
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM),-CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-C-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, - CH₂C₄H₆-*p*-NO₂, -CH₂CeH₃-2,6-Cl₂, -CH₂C₆H₃-3,4-Cl₂, -CH₂-9-anthyl, -CH₂-4-pyridyl, Si(CH₃)₃, -Si(CH₃)₂C(CH₃)₃;
und wobei R¹, R², R ³ R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

9. Verfahren nach Anspruch 7, wobei als Edukt eine Verbindung mit einer Struktur gemäß Formel **20** eingesetzt wird 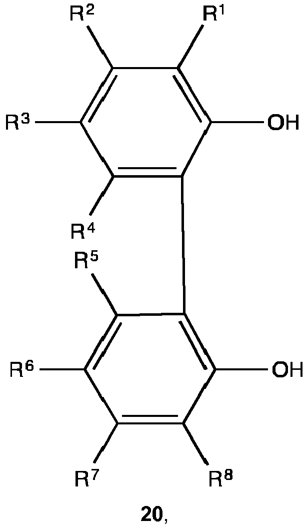
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
und wobei die Phosphor und Chlor enthaltende Verbindung ein Chlorophosphit ist, das eine Struktur gemäß Formel 50 aufweist 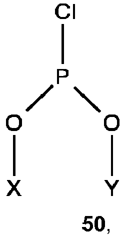 wobei X und Y jeweils unabhängig voneinander ausgewählt sind aus: -(C₁-C₁₂)-Alkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, - (C₁-C₁₂)-Alkyl-(C₆-C₂₀)-Aryl, -(C₆-C₂₀)-Aryl-COO-(C₁-C₁₂)-Alkyl, -(C₆-C₂₀)-Aryl-CONH-(C₁-C₁₂)-Alkyl, -(C₆-C₂₀)-Aryl-CON[(C₁-C₁₂)-Alkyl]₂, -(C₄-C₂₀)-Heteroaryl, -(C₄-C₂₀)-Heteroaryl-(C₁-C₁₂)-Alkyl, -(C₅-C₈)-Cycloalkyl, -(C₅-C₈)-Heterocycloalkyl, -(C₆-C₂₀)-Aryl-CO-(C₆-C₂₀)-Aryl.

10. Verfahren nach Anspruch 7, wobei als Edukt eine Verbindung mit einer Struktur gemäß Formel **20** eingesetzt wird 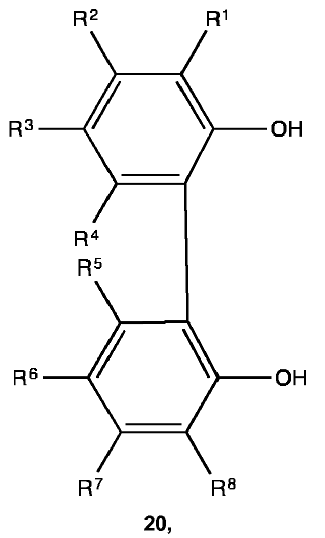
wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂;
und wobei die Phosphor und Chlor enthaltende Verbindung ein Chlorophosphit ist, das eine Struktur gemäß Formel **60** aufweist 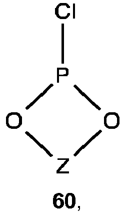 wobei Z ausgewählt ist aus: -(C₁-C₁₂)-Alkyl-, -(C₆-C₂₀)-Aryl-, -(C₆-C₂₀)-Aryl-(C₁-C₁₂)-Alkyl-, -(C₁-C₁₂-Alkyl-(C₆-C₂₀-Aryl-, -(C₄-C₂₀)-Heteroaryl-, -(C₆-C₂₀)-Aryl-CO-(C₆-C₂₀)-Aryl-, -(C₆-C₂₀)-Aryl-(C₆-C₂₀)-Aryl-.

11. Verfahren nach einem der Ansprüche 7 bis 10, wobei ein Zwischenprodukt mit einer Struktur gemäß Formel 30 gebildet wird 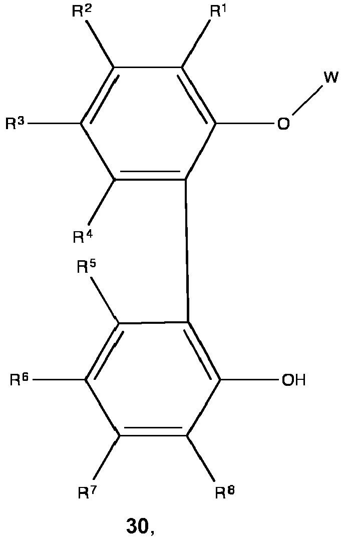
wobei W ausgewählt ist aus: -(C₁-C₁₂)-Alkyl, -Me, -CH₂CH₃, -CH₂CH₂CH₃, -CH₂OCH₃ (MOM), -CH₂OCH₂C₆H₅ (BOM), -CH₂OCH₂CH₂OCH₃ (MEM), -CH₂OCH₂CH₂Si(CH₃)₃ (SEM), -CH₂SCH₃ (MTM), -CH₂SC₆H₅ (PTM),-CH₂CN, -CF₂CHCl₂, -CH₂CH₂Cl, -CH₂CH₂Br, -2-tetrahydropyranyl, -CH(OC₂H₅)CH₃, -CH₂COC₆H₅, -CH₂COC₆H₄-4-Br, -CH₂-C-C₃H₅, -CH₂CH=CH₂, -CH₂CH=C(CH₃)₂, -CH(CH₃)₂, -*c*-C₆H₁₁, -C(CH₃)₃, -CH₂C₆H₅, -CH₂C₆H₃-2,4-(CH₃)₂, -CH₂C₆H₄-4-OCH₃, -CH₂C₆H₄-*o*-NO₂, - CH₂C₄H₆-*p*-NO₂, -CH₂CeH₃-2,6-Cl₂, -CH₂C₆H₃-3,4-Cl₂, -CH₂-9-anthyl, -CH₂-4-pyridyl, Si(CH₃)₃, -Si(CH₃)₂C(CH₃)₃;
und wobei R¹, R², R³, R⁴, R⁵, R⁶, R⁷, R⁸ jeweils unabhängig voneinander ausgewählt sind aus: -H, -(C₁-C₁₂)-Alkyl, -O-(C₁-C₁₂)-Alkyl, -O-(C₆-C₂₀)-Aryl, -(C₆-C₂₀)-Aryl, -Cl, -F, -Br, -I, -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(CrC₁₂)-Alkyl, -CO-(C₆-C₂₀)-Aryl, -COOH, -OH, -SO₃H, -SO₃Na, -NO₂, -CN, -NH₂, -N[(C₁-C₁₂)-Alkyl]₂.

12. Komplex, umfassend ein Metallatom oder -ion und zumindest eine Verbindung nach einem der Ansprüche 1 bis 6.

13. Komplex nach Anspruch 12, wobei das Metallatom oder -ion ausgewählt ist aus der Gruppe, die Rh, Ru, Co und Ir umfasst.

14. Verwendung einer Verbindung nach einem der Ansprüche 1 bis 6 oder eines Komplexes nach einem der Ansprüche 12 oder 13 in der Hydroformylierung von (C₂-C₂₄)-Olefinen.

15. Verwendung nach Anspruch 14, wobei die Hydroformylierungsreaktion die folgenden Schritte umfasst:
a) Vorlegen eines Olefins,
b) Zugabe eines Komplexes nach einem der Ansprüche 12 oder 13, oder einer Verbindung nach einem der Ansprüche 1 bis 6 sowie einer ein Metallatom oder Metallion enthaltenden Verbindung,
c) Zuführen von H₂ und CO,
d) Erwärmen des Reaktionsgemisches, wobei das Olefin zu einem Aldehyd umgesetzt wird.
